# EUROPÄISCHE PATENTANMELDUNG

(11) **EP 1 847 253 A1**
(43) Veröffentlichungstag der Anmeldung: **24.10.2007**
(21) Anmeldenummer: 07007692.2
(22) Anmeldetag: 16.04.2007
(51) Int. Cl.: A61K 8/81, A61Q 5/06, A61Q 17/04, A61Q 19/00, A61Q 19/08, A61Q 19/10, A61K 9/00, C08F 291/00, C08L 51/00

(54) **Wasserlösliche Polymere und ihre Verwendung in kosmetischen und pharmazeutischen Zubereitungen**

(30) Priorität: 21.04.2006 DE 102006018523
(71) Anmelder: CLARIANT INTERNATIONAL LTD., 4132 Muttenz (CH)
(72) Erfinder: Milbradt, Robert, Dr., 65191 Wiesbaden (DE); Mildner, Carina, 65931 Frankfurt am Main (DE)
(74) Vertreter: Hütter, Klaus

(57) **Zusammenfassung**

Es werden kosmetische und pharmazeutische Zubereitungen beschrieben, enthaltend
I) ein oder mehrere wasserlösliche unvernetzte Copolymere, enthaltend
A) eine oder mehrere Struktureinheiten der Formel (1) 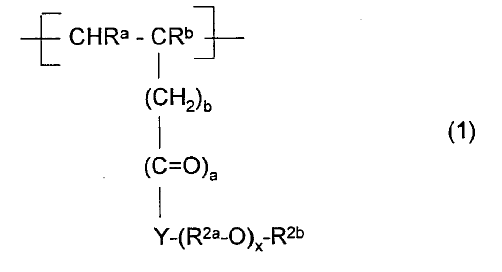 und
B) eine oder mehrere Struktureinheiten der Formel (2) 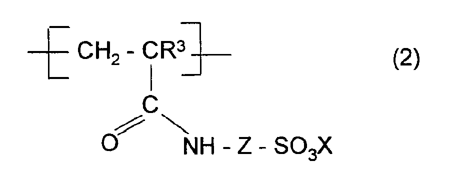 und
II) ein oder mehrere wasserlösliche oder wasserquellbare vernetzte oder unvernetzte copolymere oder homopolymere Verdicker.

## Beschreibung

Die vorliegende Erfindung betrifft eine Mischung aus unvernetzten Polymeren, die durch Copolymerisation von Makromonomeren und Comonomeren auf Basis von Acryloyldimethyltaurinsäure bzw. deren Salze hergestellt wurden und mindestens einem weiteren wasserlöslichen oder wasserquellbaren polymeren Verdicker, und die Verwendung der Polymermischung in kosmetischen und pharmazeutischen Mitteln.

Verbraucherwünsche und Rheologie kosmetischer Produkte sind eng miteinander verknüpft. So wird. z.B. das visuelle Erscheinungsbild einer Creme oder Lotion durch die Viskosität beeinflusst. Die sensorischen Eigenschaften, wie Konsistenz oder Verteilbarkeit bestimmen das individuelle Profil eines Kosmetikproduktes. Auch die Effektivität von Wirksubstanzen (z.B. Sonnenschutzfilter) und auch die Lagerstabilität der Formulierung stehen in engem Zusammenhang mit den rheologischen Eigenschaften der Produkte.

Im kosmetischen Bereich kommt Polyelektrolyten als Verdicker und Gelbildner eine tragende Rolle zu. Stand der Technik sind insbesondere Verdicker auf Basis der Poly(meth)acrylsäure und deren wasserlöslichen Copolymeren. Die Vielfalt der möglichen Strukturen und die damit verbundenen vielfältigen Anwendungsmöglichkeiten drücken sich nicht zuletzt in einer Vielzahl von Patenten aus, die seit Mitte der 70iger Jahre weltweit angemeldet wurden.

Ein wesentlicher Nachteil der Verdicker auf Basis von Poly(meth)acrylsäure ist die starke pH-Abhängigkeit der Verdickungsleistung. So wird im Allgemeinen eine hinreichend hohe Viskosität nur dann aufgebaut, wenn der pH Wert der Formulierung oberhalb von pH 6 eingestellt ist, d.h. die Poly(meth)acrylsäure in neutralisierter Form vorliegt. Ferner sind die entsprechenden Mittel empfindlich gegenüber UV-Strahlung und auch Scherung und vermitteln zudem auf der Haut ein klebriges Gefühl. Ebenso ist die Handhabung derartiger Verdicker problematisch. Da die Verdicker im Allgemeinen in saurer Form vorliegen, bedarf es bei der Formulierung eines zusätzlichen Neutralisationschrittes.

In den 90iger Jahren wurden neuartige Verdicker auf Basis vernetzter und neutralisierter Polyacryloyldimethyltaurate in den Markt eingeführt (EP-B-0 815 828, EP-B-0 815 844, EP-B-0 815 845 und EP-A-0 850 642). Sowohl in Form des vorneutralisierten Homopolymers als auch als korrespondierendes Copolymer (Aristoflex^{®} AVC, Clariant GmbH) sind diese Verdicker den Poly(meth)acrylat-Typen in vieler Hinsicht überlegen. Beispielsweise zeigen Acryloyldimethyltaurat-basierende Verdickersysteme hervorragende Eigenschaften in pH-Bereichen unterhalb von pH 6, also in einem pH-Bereich, in dem mit herkömmlichen Poly(meth)acrylat-Verdickern nicht mehr gearbeitet werden kann. Hohe UV- und Scherstabilität, hervorragende viskoelastische Eigenschaften, leichte Verarbeitbarkeit und ein günstiges toxikologisches Profil des Hauptmonomeren machen Acryloyldimethyltaurat-basierende Verdickersysteme zu modernen, neuen Vertretern mit hohem Potenzial für die Zukunft.

Im Laufe der letzten Jahre etablierte sich ein weiteres Verdickerkonzept auf dem Markt. Hierbei wurde durch hydrophobe Modifikation der konventionellen Poly(meth)acrylate der Zugang zu Polymeren gefunden, die sowohl verdickende als auch emulgierende/dispergierende Eigenschaften aufweisen. Beispiele für kommerzielle hydrophob modifizierte Poly(meth)acrylate sind Pemulen^{®} TR-1 und TR-2 von BF-Goodrich und Aculyn^{®} 22 Rohm und Haas. Da diese hydrophob modifizierten Polymere auf der Basis von (Meth)acrylsäure aufgebaut sind, besitzen sie auch die oben erwähnten Nachteile der Poly(meth)acrylate.

In EP 1 069 142 werden hydrophob modifizierte Copolymere auf Basis von Acryloyldimethyltaurinsäure bzw. deren Salze und deren Verwendung als Verdicker, Dispergiermittel, Suspendiermittel, Emulgator, Stabilisator und Konsistenzgeber beschrieben. Während die unvernetzten Copolymere dieser Polymerklasse nur geringes Verdickungsvermögen zeigen und zur Fadenbildung neigen, sind die vernetzten Typen hervorragende Verdicker. Nachteilig ist, dass die Viskosität der mit vernetzten Acryloyldimethyltaurinsäure-Copolymeren verdickten Formulierungen in Gegenwart von Elektrolyten stark abfällt und die Gelstruktur bricht.

Es bestand die Aufgabe, Substanzen für kosmetische, pharmazeutische und dermatologische Zubereitungen zur Verfügung zu stellen, die gute verdickende und konsistenzgebende Eigenschaften und gleichzeitig hohe Elektrolytstabilität zeigen, mit wässrigen Systemen und mit Ölsystemen, sowie mit Inhaltsstoffen kosmetischer Produkte, wie Tensiden gut verträglich sind, ein klares visuelles Erscheinungsbild haben, leicht verarbeitbar und kompatibel mit Wirksubstanzen (z.B. Sonnenschutzfiltern) sind, Temperatur- und Lagerbeständigkeit aufweisen, aber auch hautverträglich und toxikologisch unbedenklich sind.

Es wurde überraschend gefunden, dass diese Aufgabe gelöst wird durch eine Kombination von unvernetzten Copolymeren auf Basis von Acryloyldimethyltaurinsäure bzw. deren Salze mit mindestens einem weiteren wasserlöslichen oder wasserquellbarem polymeren Verdicker.

Durch den Einsatz einer Mischung aus unvernetzten Copolymeren auf Basis von Acryloyldimethyltaurinsäure bzw. deren Salze, vorzugsweise aus den entsprechenden unvernetzten hydrophob modifizierten Copolymeren, mit mindestens einem weiteren wasserlöslichen oder wasserquellbarem polymeren Verdicker lassen sich insbesondere in wässrigen Systemen elektrolytstabile Gele mit guter pH-Stabilität, guter Hautsensorik, geringer Klebrigkeit und stabiler Konsistenz herstellen. Die Copolymermischung eignet sich hervorragend als Konsistenzgeber und Verdicker, insbesondere von Hair-Stylingmitteln.

Gegenstand der Erfindung sind kosmetische oder pharmazeutische Zubereitungen, enthaltend
I) ein oder mehrere wasserlösliche unvernetzte Copolymere, enthaltend
   A) eine oder mehrere Struktureinheiten der Formel (1) 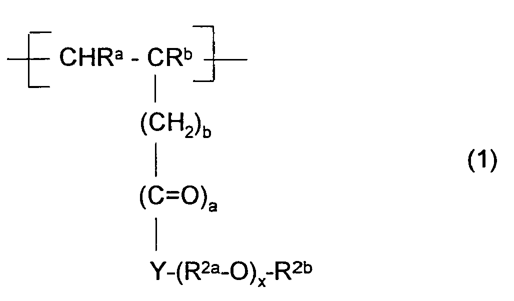 worin
      - R^{a}: H oder CH₃;
      - R^{b}: H oder CH₃;
      - a: 0 oder 1;
      - b: 0 oder 1;
      - Y: O, S, PH oder NH;
      - R^{2a}: eine lineare oder verweigte (C₂-C₄)-Alkylengruppe;
      - x: eine ganze Zahl zwischen 1 und 500; und
      - R^{2b}: Wasserstoff oder einen gesättigten oder ein- oder mehrfach ungesättigten linearen oder verzweigten aliphatischen, cycloaliphatischen oder aromatischen (C₁-C₃₀)-Kohlenwasserstoffrest bedeuten und
   B) eine oder mehrere Struktureinheiten der Formel (2) 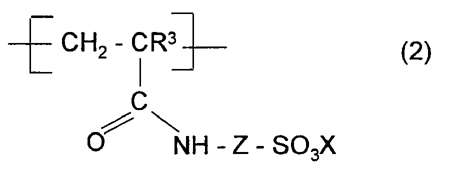 worin
      - R³: für Wasserstoff, Methyl oder Ethyl steht,
      - Z: für (C₁-C₈)-Alkylen steht, und
      - X: für Wasserstoff, Lithium, Natrium, Kalium, Magnesium, Calcium, Ammonium, Monoalkylammonium, Dialkylammonium, Trialkylammonium oder Tetraalkyl-ammonium, wobei die Alkylsubstituenten der Ammoniumionen unabhängig voneinander (C₁-C₂₂)-Alkylreste darstellen, die mit 0 bis 3 Hydroxyalkylgruppen
      besetzt sein können, deren Alkylkettenlänge in einem Bereich von C₂ bis C₁₀ variieren kann, steht oder X auch für ein bis dreifach ethoxylierte Ammoniumverbindungen mit gleichem oder unterschiedlichem Ethoxylierungsgrad steht, und wobei in den unvernetzten Copolymeren auch Struktureinheiten der Formel (2) mit unterschiedlichem X enthalten sein können,
      und
II) ein oder mehrere wasserlösliche oder wasserquellbare vernetzte oder unvernetzte copolymere oder homopolymere Verdicker.

Bei den Struktureinheiten der Formel (1) handelt es sich vorzugsweise um Struktureinheiten, die aus Makromonomeren hervorgegangen sind.

Makromonomere im Sinne der vorliegenden Erfindung sind polymerisierbare chemische Verbindungen, die mindestens eine olefinische Doppelbindung tragen und die in einer Polymerisationsreaktion zu Struktureinheiten der Formel (1) führen, wobei die Variable x in der Wiederholungseinheit (R^{2a} -O)ₓ im Durchschnitt einen Wert größer 1 hat.

Innerhalb einer Struktureinheit der Formel (1) kann (R^{2a} -O) auch unterschiedliche Bedeutungen annehmen.

In einer bevorzugten Ausführungsform der Erfindung sind R^{a}, R^{b}, a und b in der Struktureinheit der Formel (1) aus folgenden Kombinationen ausgewählt:
R^{a} = R^{b} = H und a = b = 0;
R^{a} = R^{b} = H, a = 0 und b = 1;
R^{a} = R^{b} = H, a = 1 und b = 0; oder
R^{a} = H, R^{b} = CH₃, a = 1 und b = 0.

In einer besonders bevorzugten Ausführungsform der Erfindung sind R^{a}, R^{b}, a und b in der Struktureinheit der Formel (1) aus folgenden Kombinationen ausgewählt:
R^{a} = R^{b} = H, a = 1 und b = 0 oder
R^{a} = H, R^{b} = CH₃, a = 1 und b = 0.

In einer weiteren bevorzugten Ausführungsform der Erfindung steht in der Struktureinheit der Formel (1)
- R^{2a}: für einen Ethylen- oder Propylenrest, vorzugsweise für einen Ethylenrest,
- x: für eine Zahl zwischen 3 und 50, vorzugsweise zwischen 6 und 30, und
- R^{2b}: für einen gesättigten oder einen ein- oder mehrfach ungesättigten aliphatischen oder cycloaliphatischen Kohlenwasserstoffrest.

In einer weiteren bevorzugten Ausführungsform der Erfindung steht R^{2b} in der Struktureinheit der Formel (1) für einen (C₆-C₂₂)-Kohlenwasserstoffrest, vorzugsweise für einen (C₁₂-C₁₈)-Kohlenwasserstoffrest. Besonders bevorzugt ist dieser Kohlenwasserstoffrest ein Alkyl- oder ein ein- oder mehrfach ungesättigter Alkenylrest, vorzugsweise ein Alkylrest

In einer weiteren bevorzugten Ausführungsform der Erfindung steht R^{2b} in der Struktureinheit der Formel (1) für einen Rest ausgewählt aus Stearyl, Lauryl, Cocoyl, Undecyl, Behenyl, Cetearyl, Cetyl und Myristyl und vorzugsweise für einen Rest ausgewählt aus Stearyl, Lauryl, Cetyl und Myristyl.

In einer weiteren bevorzugten Ausführungsform der Erfindung steht in der Struktureinheit der Formel (2) R³ für H, Z für -C(CH₃)₂-CH₂- und X für Wasserstoff, Natrium, Kalium oder Ammonium, vorzugsweise für H oder Ammonium, wobei in den unvernetzten Copolymeren auch Struktureinheiten der Formel (2) mit unterschiedlichem X enthalten sein können.

In einer weiteren bevorzugten Ausführungsform der Erfindung beträgt der Neutralisierungsgrad der Struktureinheit der Formel (2) 70 bis 100 mol-%, vorzugsweise 80 bis 100 mol-% und besonders bevorzugt 80 bis 99 mol-%.

In einer weiteren bevorzugten Ausführungsform der Erfindung betragen die molaren Anteile der Struktureinheit der Formel (1) und der Struktureinheit der Formel (2) im Copolymer der Komponente I) jeweils von 0,1 bis 99,9 mol-%.

In einer besonders bevorzugten Ausführungsform der Erfindung beträgt der Anteil der Struktureinheit der Formel (1) im Copolymer der Komponente I) von 50,1 bis 99,9 mol-%, bevorzugt von 70 bis 95 mol-% und besonders bevorzugt von 80 bis 90 mol-%.

In einer anderen besonders bevorzugten Ausführungsform der Erfindung beträgt der Anteil der Struktureinheit der Formel (1) im Copolymer der Komponente I) von 0,1 bis 50 mol-%, bevorzugt von 5 bis 25 mol-% und besonders bevorzugt von 6 bis 15 mol-%.

Weiterhin bevorzugte erfindungsgemäße kosmetische oder pharmazeutische Zubereitungen sind solche, enthaltend
I) ein oder mehrere wasserlösliche unvernetzte Copolymere, herstellbar durch radikalische Copolymerisation von
   A) einem oder mehreren Makromonomeren der Formel (1a)

      R¹¹-Y-(R²¹-O)ₓ-R³¹ (1a)

      worin R¹¹ einen Vinyl-, Allyl-, Acryl- oder Methacrylrest; R²¹ (C₂-C₄)-Alkylen; x eine ganze Zahl zwischen 1 und 500; Y = O, S, PH oder NH; und R³¹ Wasserstoff oder einen gesättigten oder ungesättigten linearen oder verzweigten aliphatischen, cycloaliphatischen oder aromatischen (C₁-C₃₀)-Kohlenwasserstoffrest bedeuten und
   B) einem oder mehreren Monomeren der Formel (2a) 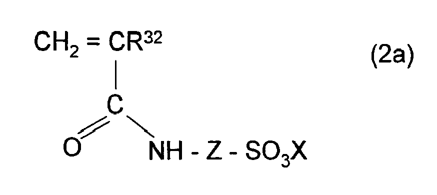
   worin R³² Wasserstoff, Methyl oder Ethyl, Z (C₁-C₈)-Alkylen und X für Wasserstoff, Lithium, Natrium, Kalium, Magnesium, Calcium, Ammonium, Monoalkylammonium, Dialkylammonium, Trialkylammonium oder Tetraalkyl-ammonium, wobei die Alkylsubstituenten der Ammoniumionen unabhängig voneinander (C₁-C₂₂)-Alkylreste darstellen, die mit 0 bis 3 Hydroxyalkylgruppen besetzt sein können, deren Alkylkettenlänge in einem Bereich von C₂ bis C₁₀ variieren kann, steht, oder X auch für ein bis dreifach ethoxylierte Ammoniumverbindungen mit unterschiedlichem Ethoxylierungsgrad steht und wobei in den unvernetzten Copolymeren auch Struktureinheiten hervorgegangen aus den Monomeren der Formel (2a) mit unterschiedlichem X enthalten sein können und
II) ein oder mehrere wasserlösliche oder wasserquellbare vernetzte oder unvernetzte copolymere oder homopolymere Verdicker.

In den Verbindungen der Formel (1a) steht R¹¹ bevorzugt für einen Acryl- oder Methacrylrest.

In den Verbindungen der Formel (1a) steht R²¹ bevorzugt für einen Ethylen- oder Propylenrest.

In den Verbindungen der Formel (1a) steht x bevorzugt für eine Zahl zwischen 3 und 50, besonders bevorzugt für eine Zahl zwischen 6 und 30.

In den Verbindungen der Formel (1a) steht R³¹ bevorzugt für aliphatische oder cycloaliphatische Kohlenwasserstoffe, die gesättigt oder ungesättigt sein können, besonders bevorzugt für einen (C₆-C₂₂)-Kohlenwasserstoffrest, insbesondere bevorzugt für einen (C₁₂-C₁₈)-Kohlenwasserstoffrest. Außerordentlich bevorzugt bedeutet R³¹ einen Alkyl- oder einen ein- oder mehrfach ungesättigten Alkenylrest, hierunter bevorzugt ist wiederum der Alkylrest. Ganz außerordentlich bevorzugt steht R³¹ für einen Rest ausgewählt aus Stearyl, Lauryl, Cocoyl, Undecyl, Behenyl, Cetearyl, Cetyl und Myristyl und hierunter bevorzugt wiederum für einen Rest ausgewählt aus Stearyl, Lauryl, Cetyl und Myristyl.

In den Verbindungen der Formel (2a) steht R³² bevorzugt für H, Z bevorzugt für -C(CH₃)₂-CH₂- und X bevorzugt für Wasserstoff, Natrium, Kalium oder Ammonium, besonders bevorzugt für Wasserstoff oder Ammonium, wobei in den unvernetzten Copolymeren auch Struktureinheiten der Formel (2a) mit unterschiedlichem X enthalten sein können.

Vorzugsweise ist der Neutralisierungsgrad der Struktureinheit hervorgegangen aus den Monomeren der Formel (2a) von 70 bis 100 mol-%, vorzugsweise von 80 bis 100 mol-% und besonders bevorzugt von 80 bis 99 mol-%.

In einer weiteren bevorzugten Ausführungsform der Erfindung betragen die molaren Anteile der Struktureinheit hervorgegangen aus den Makromonomeren der Formel (1a) und der Struktureinheit hervorgegangen aus den Monomeren der Formel (2a) im Copolymer der Komponente I) jeweils von 0,1 bis 99,9 mol-%.

In einer besonders bevorzugten Ausführungsform der Erfindung beträgt der Anteil der Struktureinheit hervorgegangen aus den Makromonomeren der Formel (1a) im Copolymer der Komponente I) von 50,1 bis 99,9 mol-%, bevorzugt von 70 bis 95 mol-% und besonders bevorzugt von 80 bis 90 mol-%.

In einer anderen besonders bevorzugten Ausführungsform der Erfindung beträgt der Anteil der Struktureinheit hervorgegangen aus den Makromonomeren der Formel (1a) im Copolymer der Komponente I) von 0,1 bis 50 mol-%, bevorzugt von 5 bis 25 mol-% und besonders bevorzugt von 6 bis 15 mol-%.

Bevorzugte wasserlösliche oder wasserquellbare vernetzte oder unvernetzte copolymere oder homopolymere Verdicker der Komponente II) sind ausgewählt aus
a) Polymeren auf Basis von Methacrylsäure oder Acrylsäure und modifizierter (Meth)acrylsäure, vorzugsweise vernetzte Polymere der Acrylsäure wie sie unter den Handelsnamen Carbopol 980, 981, 954, 2984 und 5984 (CTFA Name: Carbomer) oder Synthalen M und Synthalen K erhältlich sind, Copolymere aus (Meth)acrylsäure und Polyalkylenpolyether, hydrophob modifizierte Poly(meth)acrylate, beispielsweise die unter Pemulen^{®} TR-1 und TR-2 von BF-Goodrich, Carbopol^{®} ETD 2020 von BF-Goodrich (Acrylate/C10-30 Alkyl Acrylat Polymer), Aculyn^{®} 22 von Rohm und Haas (Acrylates/Steareth-20 Methacrylate Copolymer), Aculyn^{®} 28 von Rohm und Haas (Acrylates/Beheneth-25 Methacrylate Copolymer), Synthalen^{®} W 2000 von 3V Sigma (Acrylate/Palmeth-25 Acrylat Copolymer), Structure^{®} 3001 von National Starch (Acrylates/Ceteth-20 Itaconat Copolymer) erhältlichen Copolymere,
b) Homopolymeren der Dimethylaminoethyl(meth)acrylate, quaternisiert mit Methylchlorid, wie unter den Handelsnamen Salcare^{®} 95 und Salcare^{®} 96 von Ciba erhältlich,
c) Copolymeren von Dimethylaminoethyl(meth)acylat, quaternisiert mit Methylchlorid und Acrylamid, wie unter den Handelsnamen Salcare^{®} SC92 oder PAS 5194, erhältlich,
d) vernetzten Copolymeren von Vinylisodecanoat und (Meth)acrylsäure, wie unter dem Handelsnamen Stabylen^{®} 30 erhältlich,
e) Polyvinylalkoholen,
f) Polyvinylmethylethern,
g) Polyacrylamiden,
h) Polyvinylamiden,
i) Polyvinylpyrrolidon,
j) Poly(meth)acrylsäuren, Poly(meth)acrylsäureestern und weiteren Poly(meth)acrylsäurederivaten,
k) Polyethylenoxiden,
l) Copolymeren aus Maleinsäureanhydrid und Vinylmethylether,
m) Polysulfonsäuren, vorzugsweise Copolymerisaten auf Basis der Acrylamido-alkylsulfonsäure und/oder deren Salzen und einem oder mehreren Comonomeren ausgewählt aus cyclischen N-Vinylcarbonsäureamiden und linearen N-Vinylcarbonsäureamiden oder auch hydrophob modifizierten vernetzten Acrylamido-alkylsulfonsäure-Copolymerisaten (z.B. wie in DE 10 059 826 beschrieben),
n) vernetzten Homopolymeren der Acrylamido-alkylsulfonsäure und/oder deren Salzen,
o) Copolymeren aus der Acrylamido-alkylsulfonsäure und/oder deren Salzen und Comonomeren ausgewählt aus Acrylamid, Hydroxyethyl(meth)acrylat und kationisch modifizierte (Meth)acrylate und
p) natürlichen und modifizierten natürlichen Polymeren auf Basis von Polysacchariden, vorzugsweise Celluloseether, Cellulosederivate, Carboxymethylcellulose, Hydroxyethylcellulose, Gelatine, Stärke und Stärkederivate, Natriumalginate, Xanthan, Guar und Guarderivate, Scleroglucan,Traganth oder Dextrinderivate, insbesondere Dextrinester.

Besonders bevorzugte wasserlösliche oder wasserquellbare vernetzte oder unvernetzte copolymere oder homopolymere Verdicker der Komponente II) sind ausgewählt aus den obengenannten Gruppen a), g), j), m), n), o) und p).

In einer weiteren bevorzugten Ausführungsform der Erfindung beträgt der Gewichtsanteil an vernetzenden Comonomeren, bezogen auf die Gesamtmasse der Polymere der Komponente II) von 0 bis 20 Gew.-%.

In einer weiteren bevorzugten Ausführungsform der Erfindung enthalten die kosmetischen oder pharmazeutischen Zubereitungen ein oder mehrere wasserlösliche unvernetzte Copolymere der Komponente I) auf Basis von Acryloyldimethyltaurinsäure bzw. deren Salzen, vorzugsweise entsprechende unvernetzte hydrophob modifizierte Copolymere der Komponente I), und ein oder mehrere wasserlösliche oder wasserquellbare vernetzte copolymere Verdicker der Komponente II) auf Basis von Acryloyldimethyltaurinsäure bzw. deren Salzen, vorzugsweise entsprechende vernetzte hydrophob modifizierte copolymere Verdicker der Komponente II), wobei die unvernetzten Copolymere der Komponente I) und die vernetzten Copolymere der Komponente II) jeweils herstellbar sind durch radikalische Copolymerisation von
A) einem oder mehreren Makromonomeren der Formel (1a)

   R¹¹-Y- (R²¹-O)ₓ-R³¹ (1a)

   worin R¹¹ einen Vinyl-, Allyl-, Acryl- oder Methacrylrest; R²¹ (C₂-C₄)-Alkylen; x eine ganze Zahl zwischen 1 und 500; Y = O, S, PH oder NH; und R³¹ Wasserstoff oder einen gesättigten oder ungesättigten linearen oder verzweigten aliphatischen, cycloaliphatischen oder aromatischen (C₁-C₃₀)-Kohlenwasserstoffrest bedeuten
   und
B) einem oder mehreren Comonomeren der Formel (2a) 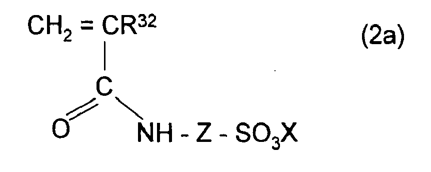 worin R³² Wasserstoff, Methyl oder Ethyl, Z (C₁-C₈)-Alkylen und X ein Wasserstoff, ein Ammonium-, Alkali- oder Erdalkali-lon bedeuten kann und wobei in den unvernetzten Copolymeren der Komponente I) und den vernetzten Copolymeren der Komponente II) auch Struktureinheiten hervorgegangen aus den Monomeren der Formel (2a) mit unterschiedlichem X enthalten sein können
   und nur im Fall der vernetzten Copolymere der Komponente II)
C) zusätzlich einem oder mehreren Vernetzern.

Diese Schreibweise bedeutet im Rahmen der vorliegenden Erfindung, dass die Copolymere der Komponente I) herstellbar sind durch radikalische Copolymerisation der Monomere der Formeln (1a) und (2a) und die Copolymere der Komponente II) herstellbar sind durch radikalische Copolymerisation der Monomere der Formeln (1a) und (2a) und zusätzlich einem oder mehreren Vernetzern.

Bei den Vernetzern der Gruppe C) handelt es sich um Monomere mit zwei oder mehr Doppelbindungen.

In den Verbindungen der Formel (1a) steht R¹¹ bevorzugt für einen Acryl- oder Methacrylrest.

In den Verbindungen der Formel (1a) steht R²¹ bevorzugt für einen Ethylen- oder Propylenrest.

In den Verbindungen der Formel (1a) steht x bevorzugt für eine Zahl zwischen 3 und 50, besonders bevorzugt für eine Zahl zwischen 6 und 30.

In den Verbindungen der Formel (1a) steht R³¹ bevorzugt für aliphatische oder cycloaliphatische Kohlenwasserstoffe, die gesättigt oder ungesättigt sein können, besonders bevorzugt für einen (C₆-C₂₂)-Kohlenwasserstoffrest, insbesondere bevorzugt für einen (C₁₂-C₁₈)-Kohlenwasserstoffrest. Außerordentlich bevorzugt bedeutet R³¹ einen Alkyl- oder einen ein- oder mehrfach ungesättigten Alkenylrest, hierunter bevorzugt ist wiederum der Alkylrest. Ganz außerordentlich bevorzugt steht R³¹ für einen Rest ausgewählt aus Stearyl, Lauryl, Cocoyl, Undecyl, Behenyl, Cetearyl, Cetyl und Myristyl und hierunter bevorzugt wiederum für einen Rest ausgewählt aus Stearyl, Lauryl, Cetyl und Myristyl.

In den Verbindungen der Formel (2a) steht R³² bevorzugt für H, Z bevorzugt für -C(CH₃)₂-CH₂- und X bevorzugt für Wasserstoff, Natrium, Kalium oder Ammonium, besonders bevorzugt für Wasserstoff oder Ammonium, wobei in den unvernetzten Copolymeren der Komponente I) und in den vernetzten Copolymeren der Komponente II) auch Struktureinheiten der Formel (2a) mit unterschiedlichem X enthalten sein können.

Vorzugsweise ist der Neutralisierungsgrad der Struktureinheit hervorgegangen aus den Monomeren der Formel (2a) von 70 bis 100 mol-%, vorzugsweise von 80 bis 100 mol-% und besonders bevorzugt von 80 bis 99 mol-%.

In einer weiteren bevorzugten Ausführungsform der Erfindung betragen die molaren Anteile der Struktureinheit hervorgegangen aus den Makromonomeren der Formel (1a) und der Struktureinheit hervorgegangen aus den Monomeren der Formel (2a) im Copolymer der Komponente I) jeweils von 0,1 bis 99,9 mol-%.

In einer weiteren bevorzugten Ausführungsform der Erfindung betragen die molaren Anteile der Struktureinheit hervorgegangen aus den Makromonomeren der Formel (1a) und der Struktureinheit hervorgegangen aus den Monomeren der Formel (2a) im Copolymer der Komponente II) jeweils von 0,1 bis 99,85 mol-% und der molare Anteil der Struktureinheit hervorgegangen aus dem einen oder den mehreren Vernetzern von 0,05 bis 8 mol-%.

In einer besonders bevorzugten Ausführungsform der Erfindung beträgt der Anteil der Struktureinheit hervorgegangen aus den Makromonomeren der Formel (1 a) im Copolymer der Komponente I) von 50,1 bis 99,9 mol-%, bevorzugt von 70 bis 95 mol-% und besonders bevorzugt von 80 bis 90 mol-%.

In einer weiteren besonders bevorzugten Ausführungsform der Erfindung beträgt der Anteil der Struktureinheit hervorgegangen aus den Makromonomeren der Formel (1a) im Copolymer der Komponente II) von 50,1 bis 99,85 mol-%, bevorzugt von 70 bis 95 mol-% und besonders bevorzugt von 80 bis 90 mol-% und der molare Anteil der Struktureinheit hervorgegangen aus dem einen oder den mehreren Vernetzern von 0,05 bis 8 mol-%.

In einer anderen besonders bevorzugten Ausführungsform der Erfindung beträgt der Anteil der Struktureinheit hervorgegangen aus den Makromonomeren der Formel (1a) im Copolymer der Komponente I) von 0,1 bis 50 mol-%, bevorzugt von 5 bis 25 mol-% und besonders bevorzugt von 6 bis 15 mol-%.

In einer weiteren besonders bevorzugten Ausführungsform der Erfindung beträgt der Anteil der Struktureinheit hervorgegangen aus den Makromonomeren der Formel (1 a) im Copolymer der Komponente II) von 0,1 bis 50 mol-%, bevorzugt von 5 bis 25 mol-% und besonders bevorzugt von 6 bis 15 mol-% und der molare Anteil der Struktureinheit hervorgegangen aus dem einen oder den mehreren Vernetzern von 0,05 bis 8 mol-%.

Weiterhin bevorzugte erfindungsgemäße Zubereitungen sind solche, worin der oder die wasserlöslichen oder wasserquellbaren vernetzten oder unvernetzten copolymeren oder homopolymeren Verdicker der Komponente II) ausgewählt sind aus Copolymeren, bestehend im Wesentlichen aus
a1) 1 bis 50 Gew.-% der wiederkehrenden Struktureinheit der Formel (3) 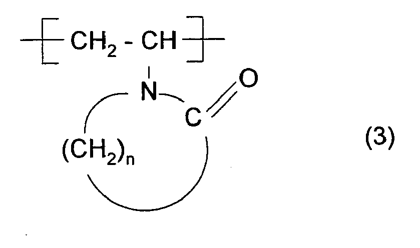 wobei n eine ganze Zahl von 2 bis 9 bedeutet
   oder
a2) 1 bis 50 Gew.-% der wiederkehrenden Struktureinheit der Formel (4) 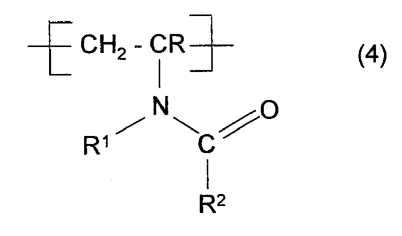 wobei R, R¹ und R² gleich oder verschieden sein können und Wasserstoff oder eine lineare oder verzweigte Alkyl- oder Alkenylgruppe mit jeweils 1 bis 30, bevorzugt 1
   bis 20, insbesondere 1 bis 12 C-Atomen, bedeuten
   oder
a3) 1 bis 50 Gew.-% einer Mischung der wiederkehrenden Struktureinheit der Formel (3) und der wiederkehrenden Struktureinheit der Formel (4)
   und
b) 49,99 bis 98,99 Gew.-% der wiederkehrenden Struktureinheit der Formel (2) 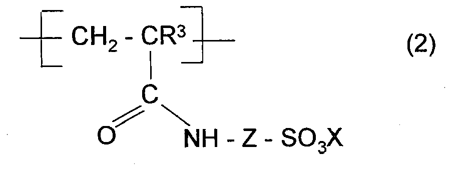 worin
   - R³: für Wasserstoff, Methyl oder Ethyl steht,
   - Z: für (C₁-C₈)-Alkylen steht, und
   - X: für Wasserstoff, Lithium, Natrium, Kalium, Magnesium, Calcium, Ammonium, Monoalkylammonium, Dialkylammonium, Trialkylammonium oder Tetraalkyl-ammonium, wobei die Alkylsubstituenten der Ammoniumionen unabhängig voneinander (C₁-C₂₂)-Alkylreste darstellen, die mit 0 bis 3 Hydroxyalkylgruppen besetzt sein können, deren Alkylkettenlänge in einem Bereich von C₂ bis C₁₀ variieren kann, steht oder X auch für ein bis dreifach ethoxylierte Ammoniumverbindungen mit gleichem oder unterschiedlichem Ethoxylierungsgrad steht, und wobei in den Copolymeren auch Struktureinheiten der Formel (2) mit unterschiedlichem X enthalten sein können, und
c) 0 bis 8 Gew.-% an vernetzenden Strukturen, die aus Monomeren mit mindestens zwei olefinischen Doppelbindungen hervorgehen.

In einer weiteren bevorzugten Ausführungsform der Erfindung ist der oder sind die wasserlöslichen oder wasserquellbaren vernetzten oder unvernetzten copolymeren oder homopolymeren Verdicker der Komponente II) ausgewählt aus vernetzten Polymeren, die vernetzende Strukturen hervorgegangen aus Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate, bevorzugt Trimethylolpropantriacrylat (TMPTA), enthalten.

Weitere bevorzugte erfindungsgemäße Zubereitungen enthalten als Komponente II) ein oder mehrere vernetzte Homopolymere, die in statistischer Verteilung zu 90 bis 99,99 Gew.-% aus Struktureinheiten hervorgegangen aus Monomeren der Formel (2a) und zu 0,01 bis 10 Gew.-% aus vernetzenden Strukturen, die aus Monomeren mit mindestens zwei olefinischen Doppelbindungen hervorgegangen sind, bestehen. Bevorzugte Vernetzer sind dabei Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate. Insbesondere bevorzugt sind die vernetzenden Strukturen hervorgegangen aus Trimethylolpropantriacrylat (TMPTA).

In einer besonders bevorzugten Ausführungsform der Erfindung ist der oder sind die wasserlöslichen oder wasserquellbaren vernetzten oder unvernetzten copolymeren oder homopolymeren Verdicker der Komponente II) ausgewählt aus vernetzten Polymeren, die vernetzende Strukturen hervorgegangen aus Trimethylolpropantriacrylat enthalten.

In einer weiteren bevorzugten Ausführungsform der Erfindung enthalten die kosmetischen, pharmazeutischen oder dermatologischen Zubereitungen ein oder mehrere unvernetzte Copolymere der Komponente I) und ein oder mehrere vernetzte Polymere der Komponente II) jeweils herstellbar durch radikalische Copolymerisation von
A) einem oder mehreren Makromonomeren der Formel (5) 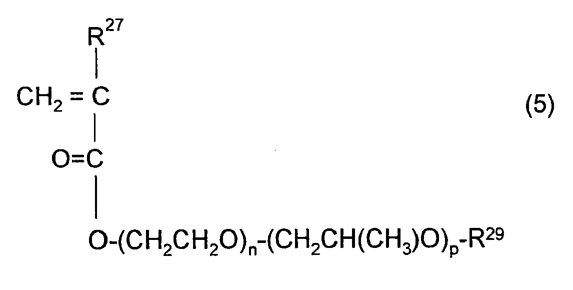 wobei R²⁷ für Wasserstoff oder für Methyl steht, R²⁹ für eine lineare oder verzweigte Alkylgruppe mit 7 bis 22, bevorzugt 8 bis 18 und besonders bevorzugt 12 bis 18 Kohlenstoffatomen, steht, die Indizes n und p unabhängig voneinander für eine Molzahl stehen und von 0 bis 30, bevorzugt von 1 bis 25 und besonders bevorzugt von 3 bis 20 variieren, unter der Voraussetzung, dass die Summe n + p größer oder gleich 1, vorzugsweise größer 1, und kleiner oder gleich 30, bevorzugt kleiner 25, besonders bevorzugt kleiner 20 und insbesondere bevorzugt kleiner 15, ist,
   und
B) einem oder mehreren Comonomeren gemäß der Formel (2a), wobei R³² für Wasserstoff, Z für -C(CH₃)₂-CH₂- und X für ein Wasserstoff-, Ammonium-, Alkali- oder Erdalkaliion, insbesondere für ein Ammonium- oder Natriumion, steht
   und nur im Fall der vernetzten Polymere der Komponente II)
C) zusätzlich Trimethylolpropantriacrylat als Vernetzer.

In einer besonders bevorzugten Ausführungsform der Erfindung enthalten die Zubereitungen ein oder mehrere unvernetzte Copolymere der Komponente I) und ein oder mehrere vernetzte Polymere der Komponente II) jeweils herstellbar durch radikalische Copolymerisation von
A) einem oder mehreren Makromonomeren, ausgewählt aus Estern der (Meth)acrylsäure mit
   (C₁₀-C₁₈)-Fettalkoholpolyglykolether mit 8 EO-Einheiten (Genapol^{®} C-080)
   C₁₁-Oxoalkoholpolyglykolether mit 8 EO-Einheiten (Genapol^{®} UD-080)
   (C₁₂-C₁₄)-Fettalkoholpolyglykolether mit 7 EO-Einheiten (Genapol^{®} LA-070)
   (C₁₂-C₁₄)-Fettalkoholpolyglykolether mit 11 EO-Einheiten (Genapol^{®} LA-110)
   (C₁₆-C₁₈)-Fettalkoholpolyglykolether mit 8 EO-Einheiten (Genapol^{®} T-080)
   (C₁₆-C₁₈)-Fettalkoholpolyglykolether mit 15 EO-Einheiten (Genapol^{®} T-150)
   (C₁₆-C₁₈)-Fettalkoholpolyglykolether mit 11 EO-Einheiten (Genapol^{®} T-110)
   (C₁₆-C₁₈)-Fettalkoholpolyglycolether mit 20 EO-Einheiten (Genapol^{®} T-200)
   (C₁₆-C₁₈)-Fettalkoholpolyglycolether mit 25 EO-Einheiten (Genapol^{®} T-250)
   (C₁₈-C₂₂)-Fettalkoholpolyglycolether mit 25 EO-Einheiten
   und/oder iso-(C₁₆-C₁₈)-Fettalkoholpolyglycolether mit 25 EO-Einheiten
   und
B) einem oder mehreren Comonomeren ausgewählt aus Acrylamidopropylmethylensulfonsäure und/oder deren Natrium- oder Ammoniumsalz
   und nur im Falle der vernetzten Polymere der Komponente II)
C) zusätzlich einem oder mehreren Vernetzern, insbesondere Trimethylolpropantriacrylat.

Bei den Genapol^{®}-Typen handelt es sich um Produkte der Firma Clariant.

In einer weiteren besonders bevorzugten Ausführungsform der Erfindung enthalten die erfindungsgemäßen Zubereitungen ein oder mehrere unvernetzte Copolymere der Komponente I) und ein oder mehrere vernetzte Polymere der Komponente II) jeweils herstellbar durch Copolymerisation von
A) Makromonomeren der Formel (5), wobei p für 0 und n für eine Zahl von 7 bis 25, R²⁷ für Methyl und R²⁹ für eine Alkylgruppe mit 12 bis 14 Kohlenstoffatomen oder 16 bis 18 Kohlenstoffatomen steht,
   und
B) einem oder mehreren Comonomeren ausgewählt aus Acrylamidopropylmethylensulfonsäure und/oder deren Salzen mit Natriumionen oder Ammoniumionen
   und nur im Falle der vernetzten Polymere der Komponente II)
C) zusätzlich einem oder mehreren Vernetzern, insbesondere Trimethylolpropantriacrylat.

In einer weiteren bevorzugten Ausführungsform der Erfindung enthalten die ein oder mehreren wasserlöslichen unvernetzten Copolymere der Komponente I) und/oder die ein oder mehreren wasserlöslichen oder wasserquellbaren vernetzten oder unvernetzten copolymeren oder homopolymeren Verdicker der Komponente II) ein oder mehrere weitere Struktureinheiten hervorgegangen aus einem oder mehreren Monomeren ausgewählt aus olefinisch ungesättigten Säuren und deren Salzen mit ein- und zweiwertigen Gegenionen, beispielsweise Styrolsulfonsäure, Vinylsulfonsäure, Vinylphosphonsäure, Allylsulfonsäure, Methallylsulfonsäure, Acrylsäure, (Meth)acrylsäure, Maleinsäure bzw. Maleinsäureanhydrid und deren Salze; Ester der Acryl- und der (Meth)acrylsäure mit aliphatischen, aromatischen oder cyclo-aliphatischen Alkoholen mit einer Kohlenstoffzahl von 1 bis 22; Ester der Acryl- und der (Meth)acrylsäure mit Alkylethoxylaten, offenkettige und cyclische N-Vinylamide (N-Vinyllactame) mit einer Ringgröße von 4 bis 9 Atomen, besonders bevorzugt N-Vinylformamid (NVF), N-Vinylmethylformamid, N-Vinyl-methylacetamid (VIMA), N-Vinylacetamid, N-Vinylpyrrolidon (NVP) und N-Vinylcaprolactam; Amide der Acryl- und der Methacrylsäure, besonders bevorzugt Acrylamid, N,N-Dimethylacrylamid, N,N-Diethylacrylamid, alkoxylierte Acryl- und Methacrylamide, wie z.B. MAPTAC und APTAC; 2-Vinylpyridin; 4-Vinylpyridin; Vinylacetat; Methacrylsäureglycidylester; Acrylnitril; Vinylchlorid; Vinylidenchlorid; Tetrafluorethylen und/oder DADMAC, enthalten.

Als Gegenionen für die Salze der olefinisch ungesättigten Säuren eignen sich bevorzugt Lithium, Natrium, Kalium, Magnesium, Calcium, Ammonium, Monoalkylammonium, Dialkylammonium, Trialkylammonium oder Tetraalkyl-ammonium, wobei die Alkylsubstituenten der Ammoniumionen unabhängig voneinander (C₁-C₂₂)-Alkylreste darstellen, die mit 0 bis 3 Hydroxyalkylgruppen besetzt sein können, deren Alkylkettenlänge in einem Bereich von C₂ bis C₁₀ variieren kann. Ebenfalls geeignet sind ein- bis dreifach ethoxylierte Ammoniumverbindungen mit unterschiedlichem Ethoxylierungsgrad. Als Gegenionen besonders bevorzugt sind Natrium und Ammonium. Der Neutralisationsgrad der olefinisch ungesättigten Säuren beträgt bevorzugt 70 bis 100 Mol-%.

Die Monomerenverteilung der Momeren A) und Comonomeren B) in den Polymeren der Komponente I) bzw. der Komponente II) kann beispielsweise alternierend, blockartig (auch Multiblock) oder auch statistisch (auch Gradient) sein.

Die in den erfindungsgemäßen Zubereitungen enthaltenen Polymere weisen im allgemeinen ein zahlenmittleres Molekulargewicht von 1000 bis 20.000.000, bevorzugt von 20.000 bis 5.000.000 und insbesondere bevorzugt von 100.000 bis 1.500.000 g/mol auf.

Die Kombination aus unvernetzten Acryloyldimethyltaurinsäure-Polymeren der Komponente I), vorzugsweise aus den entsprechenden unvernetzten hydrophob modifizierten Polymeren der Komponente I), mit vernetzten wasserlöslichen oder wasserquellbaren Copolymerisaten der Komponente II) auf Basis von Acrylamidoalkylsulfonsäuren und cyclischen N-Vinylcarbonsäureamiden und/oder linearen N-Vinylcarbonsäureamiden zeigt eine verdickende Wirkung in synergistischer Weise (s. Tabelle 1).

**Tabelle 1: Viskositäten [mPa*s, 25°C, H₂O dest. (Brookfield , 20 U/min)] einzelner Polymere und Polymer-Kombinationen (alle %-Angaben in Tabelle 1 sind Gew.-%)**

| Polymer-Gel | Wässriges Gel mit 0,5 % Aristoflex^{®} AVC | Wässriges Gel mit 0,5 % Copolymer gemäß Beispiel 3 | Wässriges Gel mit 0,5 % Aristoflex^{®} AVC und 0,1 % NaCl | Wässriges Gel mit 0,5 % Copolymer gemäß Beispiel 3 und 0,1 % NaCl | Wässriges Gel mit 0,5 % Aristoflex^{®} AVC / 0,5% Copolymer gemäß Beispiel 3 | Wässriges Gel mit 0,5 % Aristoflex^{®} AVC / 0,5% Copolymer gemäß Beispiel 3 und 0,1 % NaCl |
|---|---|---|---|---|---|---|
| Viskosität (mPas) | 17017 | ca. 20 | 85 | 28 | 2900 | 5350 |

Die Werte der Tabelle 1 belegen eine synergistische Steigerung der Viskositäten nach Elektrolytzugabe in Gegenwart der Polymer-Kombination im Vergleich zu den einzelnen Polymeren.

Die in den erfindungsgemäßen kosmetischen, pharmazeutischen und dermatologischen Zubereitungen eingesetzten Polymere der Komponente I) werden nach den in EP 1 069 142 beschriebenen Verfahren hergestellt.

In einer weiteren bevorzugten Ausführungsform der Erfindung liegt das Gewichtsverhältnis der unvernetzten Polymere der Komponente I), vorzugsweise der entsprechenden unvernetzten hydrophob modifizierten Polymere der Komponente I), zu dem einem oder den mehreren wasserlöslichen oder wasserquellbaren polymeren Verdickern der Komponente II) im Bereich von 1 bis 99 zu 99 bis 1, bevorzugt im Bereich von 10 bis 90 zu 90 bis 10, besonders bevorzugt im Bereich von 20 bis 80 zu 80 bis 20, und insbesondere bevorzugt im Bereich von 30 bis 70 zu 70 bis 30.

Die erfindungsgemäßen Zubereitungen enthalten die Polymer-Mischung der Komponenten I) und II) vorzugsweise in einer Menge von 0,1 bis 10 Gew.-%.

Bevorzugt betragen die Viskositäten der 1 Gew.-% igen wässrigen Lösungen, enthaltend mindestens ein Polymer der Komponente I) und mindestens ein Polymer der Komponente II) 500 bis 50 000 mPas, insbesondere 1 000 bis 40 000 mPas, außerordentlich bevorzugt 2000 bis 20 000 mPas bei 25°C (gemessen nach Brookfield).

Solche Polymere zeigen bereits bei Raumtemperatur eine hohe Verdickerleistung, gute Emulgiereigenschaften und gute Dispersionseigenschaften in wässriger, wässrig-alkoholischer und wässrig-tensidischer Lösung oder in Emulsionen. Des Weiteren zeigen Zubereitungen, enthaltend solche Polymere, eine gute Transparenz und eine hohe Elektrolytstabilität.

Die in den erfindungsgemäßen Zubereitungen enthaltene Mischung aus wasserlöslichen unvernetzten Polymeren der Komponente I) und wasserlöslichen vernetzten Polymeren der Komponente II) eignet sich als Verdicker und Dispergiermittel für wässrige Zubereitungen, wässrig-alkoholische und wässrigetensidische Zubereitungen und als Emulgatoren, Suspendiermittel mit verdickender Wirkung und Konsistenzgeber für Emulsionen und Suspensionen.

In einer weiteren bevorzugten Ausführungsform der Erfindung liegen die Zubereitungen in Form eines Haarbehandlungs-, Haarpflege-, Haarstyling- oder Haarreinigungsmittels vor.

In einer weiteren bevorzugten Ausführungsform der Erfindung liegen die Zubereitungen in Form eines wässrigen, gelförmigen kosmetischen oder pharmazeutischen Mittels vor.

Weitere bevorzugte Ausführungsformen der erfindungsgemäßen Zubereitungen sind Spülungen, Kuren, Sprühkuren, Lotionen, Cremes, beispielsweise Stylingcremes, Emulsionen, Gele, beispielsweise wässrige Refreshing-Gele, milde Reinigungsgele, anti-aging Gele, Sonnenschutzgele, Schäume, Mousse, Fluids und Sprays, insbesondere Haarconditioner, Schampoos, Volumenspray, Styling-Fluid, Haarschaum, Haargel, Festiger, Haarspray, Mousse, Haaröle, Haarwachse und Spitzenfluids.

In einer weiteren bevorzugten Ausführungsform der Erfindung handelt es sich bei den kosmetischen und pharmazeutischen Zubereitungen um tensidfreie Mittel, um tensidfreie Emulsionen, Gele, Sprays, Sprühschäume, Mousse oder Fluids.

In einer besonders bevorzugten Ausführungsform der Erfindung werden die Polymere der Komponenten I) und II) in versprühbare, pumpbare und verschäumbare Gele und Schäume, insbesondere in versprühbare Haargele und verschäumbare Sonnenschutzmittel eingearbeitet und bewirken eine Verbesserung des Sprühverhaltens der Mittel mit optimierter Tröpfchengrößenverteilung.

Vorteilhaft ist eine Haargelzusammensetzung, enthaltend ein oder mehrere Polymere der Komponente I) und ein oder mehrere Polymere der Komponente II) und mindestens ein haarfestigendes Polymer.

Die Viskosität der Gele beträgt vorzugsweise 100 bis 5000 mPa*s, besonders bevorzugt 200 bis 1000 mPa*s, insbesondere bevorzugt 250 bis 800 mPa*s, gemessen als dynamische Viskositätsmessung mit einem Bohlin Rheometer CS, Messkörper C25 bei einer Temperatur von 25°C und einer Schergeschwindigkeit von 50 s⁻¹.

Die Polymer-Mischung der Polymere der Komponenten I) und II) wird vorzugsweise in einer Menge von 0,1 bis 10, besonders bevorzugt von 0,2 bis 8 Gew.-% und das haarfestigende Polymer in einer Menge von vorzugsweise 0,1 bis 15, besonders bevorzugt von 0,5 bis 10 Gew.-% eingesetzt.

Das haarfestigende Polymer kann nichtionisch, anionisch, kationisch oder amphoter sein, ist aber vorzugsweise nichtionisch oder anionisch. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit in Wasser, Alkohol oder Wasser/Alkohol-Gemischen besitzen, um in dem erfindungsgemäßen Mittel in vollständig gelöster Form vorzuliegen. Unter haarfestigenden Polymeren werden solche Polymere verstanden, die bei Anwendung in 0,01 bis 15 Gew.-%iger wässriger, alkoholischer oder wässrig-alkoholischer Lösung oder Dispersion in der Lage sind, auf dem Haar eine haarfestigende Wirkung zu erzielen.

Geeignete synthetische, nichtionische haarfestigende Polymere sind Homo- oder Copolymere, die aus mindestens einem der folgenden Monomere aufgebaut sind: Vinylpyrrolidon, Vinylimidazol, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Dialkylaminoalkylmethacrylamid, Dialkylaminoalkylacrylamid, Alkylacrylat, Alkylmethacrylat, Propylenglycol oder Ethylenglycol, wobei die Alkylgruppen dieser Monomere C₁- bis C₁₈-Alkylgruppen, vorzugsweise C₁- bis C₇-Alkylgruppen, besonders bevorzugt C₁- bis C₃-Alkylgruppen sind. Geeignet sind z.B. Homopolymere des Vinylcaprolactams, des Vinylpyrrolidons oder des N-Vinylformamids. Weitere geeignete haarfestigende Polymere sind z.B. Copolymerisate aus Vinylpyrrolidon und Vinylacetat, Terpolymere aus Vinylpyrrolidon, Vinylacetat und Vinylpropionat, Terpolymere aus Vinylpyrrolidon, Vinylcaprolactam und Dialkylaminoalkyl(meth)acrylat, Terpolymere aus Vinylpyrrolidon, Vinylcaprolactam und Dialkylaminoalkyl(meth)acrylamid, Terpolymere aus Vinylpyrrolidon, Vinylimidazol und (Meth)acrylamid, Polyacrylamid, Polyvinylalkohol, sowie haarfestigende Polyethylenglycol/Polypropylenglykol Copolymere. Besonders bevorzugte nichtionische Polymere sind Polyvinylpyrrolidon und Polyvinylpyrrolidon/Vinylacetat Copolymere. Bevorzugt sind nichtionische Vinyllactam Homo- und Copolymere. Geeignete Vinyllactame sind z.B. Vinylcaprolactam und Vinylpyrrolidon. Besonders bevorzugt sind Polyvinylpyrrolidon, Polyvinylcaprolactam, Terpolymere aus Vinylpyrrolidon, Vinylimidazol und (Meth)acrylamid und Vinylpyrrolidon/Vinylacetat Copolymere. Bevorzugte Handelsprodukte sind Luviskol^{®} K 30, Luviskol^{®} K 90, Luviskol^{®} VA 37, Luviskol^{®} VA 64 und Luviset^{®} Clear.

Geeignete anionische haarfestigende Polymere können natürliche oder synthetische Homo- oder Copolymere mit Säuregruppen enthaltenden Monomereinheiten sein, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100 % in anionischer bzw. neutralisierter Form vor. Als Neutralisationsmittel können die oben genannten Neutralisationsmittel verwendet werden. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglycol oder Ethylenglycol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere C₁- bis C₁₈-Alkylgruppen, vorzugsweise C₁- bis C₇-Alkylgruppen, besonders bevorzugt C₁- bis C₃-Alkylgruppen sind.

Geeignete anionische Polymere sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure, sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte anionische Polymere sind vernetzte oder unvernetzte Vinylacetat/Crotonsäure Copolymere. Ebenso bevorzugt sind partial veresterte Copolymere zwischen Vinylmethylether und Maleinsäureanhydrid. Weitere geeignete anionische Polymere sind z.B. Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Geeignete filmbildende amphotere Polymere sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid, Alkylaminoalkylmethacrylat und zwei oder mehr Monomeren ausgewählt aus Acrylsäure, Methacrylsäure oder deren Ester, wobei die Alkylgruppen 1 bis 4 C-Atome enthalten, mindestens eines der Monomere eine Säuregruppe aufweist.

Weitere Beispiele für geeignete haarfestigende Polymere sind Copolymere von Acrylsäure, Methacrylat und Methacrylamidopropyltrimethylammoniumchlorid, Copolymere aus Acrylamidopropyltrimethylammoniumchlorid und Acrylaten, Copolymere aus Acrylamid, Acrylamidopropyltrimethylammoniumchlorid, 2-Amidopropylacrylamidsulfonat und Dimethylaminopropylamin oder Chitosane. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betain/ Acrylat Copolymer.

In einer bevorzugten Ausführungsform wird die erfindungsgemäße Zubereitung in einem wässrigen, einem alkoholischen oder in einem wässrig-alkoholischen Medium mit vorzugsweise mindestens 10 Gew.-%, besonders bevorzugt mindestens 50 Gew.-% Wasser und vorzugsweise maximal 40 Gew.-% Alkohol konfektioniert.

Als Alkohole können insbesondere die für kosmetische Zwecke üblicherweise verwendeten niederen Monoalkohole mit 1 bis 4 C-Atomen wie z.B. Ethanol und Isopropanol enthalten sein.

In einer weiteren bevorzugten Ausführungsform der Erfindung liegen die erfindungsgemäßen Zubereitungen in Form eines Haargels vor und sind vorzugsweise klare, transparente oder durchscheinende, farblose Mittel.

In einer weiteren bevorzugten Ausführungsform der Erfindung liegen die Zubereitungen in versprühbarer Form vor. Diese Zubereitungen haben besonders positive Sprüheigenschaften.

In einer besonders bevorzugten Ausführungsform der Erfindung enthalten die Zubereitungen einen oder mehrere Filmbildner. Vorzugsweise liegen sie in diesem Fall in Form von Haarpflege- und Reinigungsmitteln vor.

Bevorzugte Filmbildner sind je nach Anwendungszweck Salze der Phenylbenzimidazolsulfonsäure, wasserlösliche Polyurethane, beispielsweise C₁₀-Polycarbamylpolyglycerylester, Polyvinylalkohol, Polyvinylpyrrolidoncopolymere, beispielsweise Vinylpyrrolidon/Vinylacetatcopolymer, wasserlösliche Acrylsäurepolymere/Copolymere bzw. deren Ester oder Salze, beispielsweise Partialestercopolymere der Acryl/Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carboxyvinyl-Polymere, wie Carbomere und deren Salze, Polysaccharide, beispielsweise Polydextrose und Glucan, Vinylacetat/Crotonat, beispielsweise unter dem Handelsnamen Aristoflex^{®} A 60 (Clariant) erhältlich, sowie polymere Aminoxide, beispielsweise unter den Handelsnamen Diaformer^{®} Z-711, 712, 731, 651, 632, 772 (Mitsubishi Chemical) erhältliche Vertreter.

Die erfindungsgemäßen Haarbehandlungsmittel enthalten vorzugsweise 0,01 bis 15 Gew.-%, besonders bevorzugt 0,1 bis 10 Gew.-% und insbesondere bevorzugt 1 bis 5 Gew.-% an Filmbildnern, bezogen auf die fertigen Mittel.

In einer weiteren besonders bevorzugten Ausführungsform der Erfindung enthalten die kosmetischen, pharmazeutischen und dermatologischen Zubereitungen einen oder mehrere UV-Filter.

Als UV-Filter kommen vorzugsweise in Betracht 4-Aminobenzoesäure; 3-(4'-Trimethylammonium)benzyliden-boran-2-on-methylsulfat; 3,3,5-Trimethyl-cyclohexylsalicylat; 2-Hydroxy-4-methoxybenzophenon; 2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4`-Sulfo)-benzyliden-bornan-2-on und seine Salze; 2-Cyan-3,3-diphenyl-acrylsäure-(2-ethylhexylester); Polymer von N-[2(und 4)-(2-oxoborn-3-ylidenmethyl)benzyl]-acrylamid; 4-Methoxy-zimtsäure-2-ethyl-hexylester; ethoxyliertes Ethyl-4-amino-benzoat; 4-Methoxy-zimtsäureisoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-amino-carbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.

Die erfindungsgemäßen kosmetischen, pharmazeutischen und dermatologischen Zubereitungen enthalten UV-Filter vorzugsweise in den Mengen von 0,0001 bis 5 Gew.-%, besonders bevorzugt von 0,001 bis 2 Gew.-% und insbesondere bevorzugt von 0,01 bis 1 Gew.-%, bezogen auf die fertigen Zubereitungen.

In einer weiteren bevorzugten Ausführungsform der Erfindung enthalten die kosmetischen, pharmazeutischen und dermatologischen Zubereitungen ein oder mehrere Antioxidatien.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-paimitat) sowie Koniferylbenzoat des Benzoeharzes, Rutinsäure und deren Derivate, a-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid), Superoxid-Dismutase und die erfindungsgemäß geeigneten Derivate (Salze, Ester, Ether, Zucker, Nukleotide, Nukleoside, Peptide und Lipide) dieser genannten Stoffe.

Besonders vorteilhaft im Sinne der vorliegenden Erfindung können wasserlösliche Antioxidantien eingesetzt werden.

Die Antioxidantien können die Haare und die Haut vor oxidativer Beanspruchung schützen. Bevorzugte Antioxidantien sind dabei Vitamin E und dessen Derivate sowie Vitamin A und dessen Derivate.

Die Menge der Antioxidantien (eine oder mehrere Verbindungen) in den erfindungsgemäßen Zubereitungen beträgt vorzugsweise 0,001 bis 30 Gew.-%, besonders bevorzugt 0,05 bis 20 Gew.-% und insbesondere bevorzugt 1 bis 10 Gew.-%, bezogen auf das Gesamtgewicht der Zubereitungen.

Sofern Vitamin E und/oder dessen Derivate das oder die Antioxidantien darstellen, ist es vorteilhaft, deren jeweilige Konzentrationen aus dem Bereich von 0,001 bis 10 Gew.-%, bezogen auf das Gesamtgewicht der fertigen Zubereitung zu wählen.

Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 bis 10 Gew.-%, bezogen auf das Gesamtgewicht der Zubereitung, zu wählen.

In einer besonders bevorzugten Ausführungsform der Erfindung enthalten die kosmetischen oder pharmazeutischen Zubereitungen Antioxidantien ausgewählt aus Superoxid-Dismutase, Tocopherol (Vitamin E) und Ascorbinsäure (Vitamin C).

In weiteren erfindungsgemäßen Ausführungsformen enthalten die erfindungsgemäßen kosmetischen, pharmazeutischen und dermatologischen Zubereitungen antimikrobielle Wirkstoffe.

Bevorzugt geeignet als antimikrobielle Wirkstoffe sind Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsälze, Salicylate, Piroctose, insbesondere Zinksalze, Pyrithione und deren Schwermetallsalze, insbesondere Zinkpyrithion, Zinkphenolsulfat, Farnesol und Kombinationen dieser Wirksubstanzen.

Die erfindungsgemäßen Zubereitungen enthalten die antimikrobiellen Mittel bevorzugt in Mengen bis 50 Gew.-%, besonders bevorzugt in Mengen von 0,01 bis 10 Gew.-%, insbesondere bevorzugt in Mengen von 0,1 bis 10 Gew.-%.

Die in den erfindungsgemäßen Zubereitungen eingesetzten wasserlöslichen unvernetzten und vernetzten Polymere der Komponenten I) und (II) sind sehr gut kompatibel mit perlglanzgebenden Komponenten. Die erfindungsgemäßen Haarbehandlungsmittel können somit vorteilhafterweise perlglanzgebende Verbindungen enthalten, beispielsweise Fettsäuremonoalkanolamide, Fettsäuredialkanolamide, Monoester oder Diester von Alkylenglycol, insbesondere Ethylenglykol und/oder Propylenglykol oder dessen Oligomere mit höheren Fettsäuren, z.B. Palmitinsäure, Stearinsäure oder Behensäure oder Mischungen davon, Mono- oder Diester von Alkylenglykolen mit Fettsäuren, Fettsäuren und deren Metallsalze, Monoester oder Polyester von Glycerin mit Carbonsäuren und Ketosulfone verschiedener Art, bevorzugt Ethylenglycoldistearat und Polyethylenglycoldistearat mit ca. 3 Glykoleinheiten.

Die erfindungsgemäßen Haarbehandlungsmittel enthalten bevorzugt 0,1 bis 15, besonders bevorzugt 1 bis 10 Gew.-% an perlglanzgebenden Verbindungen.

Glitter- und Glanzeffekte der erfindungsgemäßen Zubereitungen lassen sich vorzugsweise durch Zugabe von Glimmer, colorierte Polyacrylester und Glimmer, Glimmer-Eisenoxid, Glimmer-Titanoxid und durch Pigmente erzeugen. Als Pigmente eignen sich Metalloxide, beispielsweise Eisenoxide, Titanoxid, Ultramarinblau, sowie mit kationischen Coatinghüllen modifizierte Pigmente, wie in WO 00/12053 und EP 504 066 beschrieben.

Die verdickende und konsistenzgebende Wirkung der in den erfindungsgemäßen Zubereitungen eingesetzten Polymere der Komponenten I) und II) entfaltet sich besonders vorteilhaft in Gegenwart von nichtionischischen und amphotären Tensiden.

Als nichtionische Tenside, die als waschaktive Substanzen eingesetzt werden können, kommen vorzugsweise in Betracht Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäureamidpolyethylenglykole; N-Alkoxypolyhydroxyfettsäureamid, insbesondere Fettsäure-N-methylglucamide, Saccharoseester; Polyglykolether, Alkylpolyglycoside, Phosphorsäureester (Mono-, Di- und Triphosphorsäureester ethoxyliert und nicht-ethoxyliert).

Der Gewichtsanteil der nichtionischen Tenside in den erfindungsgemäßen Zubereitungen (z.B. im Falle von Rinse-off-Produkten) liegt bevorzugt im Bereich von 1 bis 20 Gew.-%, besonders bevorzugt 2 bis 10 Gew.-%, insbesondere bevorzugt 3 bis 7 Gew.-% bezogen auf die fertige Zubereitung.

Bevorzugte Amphotenside sind: N-(C₁₂-C₁₈-Alkyl)-β-aminopropionate und N-(C₁₂-C₁₈-Alkyl)-β-iminodipropionate als Alkali- und Mono-, Di- und Trialkylammonium-Salze; N-Acylaminoalkyl-N,N-dimethyl-acetobetain, vorzugsweise N-(C₈-C₁₈-Acyl)aminopropyl-N,N-dimethylacetobetain; C₁₂-C₁₈-Alkyl-dimethyl-sulfopropyl-betain; Amphotenside auf Basis Imidazolin (Handelsname: Miranol^{®}, Steinapon^{®}), vorzugsweise das Natrium-Salz des 1-(β-Carboxy-methyloxyethyl)-1-(carboxymethyl)-2-lauryl-imidazoliniums; Aminoxide, z.B. C₁₂-C₁₈-Alkyldimethylaminoxid, Fettsäureamidoalkyl-dimethylaminoxid, Alkyltaurate, insbesondere Natriummethylcocoyltaurat (Hostapon CT, Clariant GmbH) Natriummethyllauroyltaurat, Isethionate, beispielsweise Natriumcocoylisethionat.

Der Gewichtsanteil der amphoteren Tenside beträgt bevorzugt 0,5 bis 20 Gew.-%, besonders bevorzugt 1 bis 10 Gew.-% bezogen auf die fertige Zubereitung.

In einer weiteren bevorzugten Ausführungsform sind die erfindungsgemäßen Zubereitungen Öl-in-Wasser Emulsionen mit einem Wasseranteil von 5 bis 95 Gew.-%, bevorzugt 15 bis 75 Gew.-%, besonders bevorzugt 25 bis 85 Gew.-%.

Erfindungsgemäße als Emulsionen vorliegende Zubereitungen können einen oder mehrere Emulgatoren enthalten. Diese Emulgatoren können gewählt werden aus der Gruppe der nichtionischen, anionischen, kationischen oder amphoteren Emulgatoren.

Als nichtionischer Emulgator eignen sich Anlagerungsprodukte von 2 bis 30 Mol Ethylenoxid und/oder bis zu 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; C₁₂-C₁₈-Fettsäuremono- und -diester von Anlagerungsprodukten von 1 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester wie z.B. Polyglycerinpolyricinoleat und Polyglycerinpoly-12-hydroxystearat. Bevorzugte flüssige Fettsäureester sind PEG-10 Polyglyceryl-2 Laurate und Polyglyceryl-2 Sesquiisostearate.

Als ionogene Emulgatoren eignen sich z.B. anionische Emulgatoren, wie mono-, di- oder tri-Phosphorsäureester, Seifen (z.B. Natriumstearat), Fettalkoholsulfate aber insbesondere kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Weiterhin können natürlich vorkommende Emulgatoren, unter denen Bienenwachs, Wollwachs, Lecithin und Sterole bevorzugt sind, verwendet werden.

Vorzugsweise sind Fettalkoholethoxylate gewählt aus der Gruppe der ethoxylierten Stearylalkohole, Cetylalkohole, Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethylenglycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether, Polyethylenglycol(20)cetylstearylether, Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Weiterhin ist es von Vorteil, die Polyethylglycolglycerinfettsäureester aus der Gruppe Polyethylenglycol(20)glyceryllaurat, Polyethylenglycol(6)glycerylcaprat/caprinat, Polyethylglycol(20)glyceryloleat, Polyethylenglycol(20)glycerylisostearat und Polyethylenglycol(18)glyceryloleat/cocoat zu wählen.

Unter den Sorbitanestern eignen sich besonders Polyethylenglykol (20)sorbitanmonolaurat, Polyethylenglycol (20)sorbitanmonostearat, Polyethylenglycol (20)sorbitanmonoisostearat, Polyethylenglycol (20)sorbitanmonopalmitat, Polyethylenglycol (20)sorbitanmonooleat.

Der Gewichtsanteil des oder der in den erfindungsgemäßen Zubereitungen enthaltenen Emulgators oder Emulgatoren, beträgt vorzugsweise 0,1 bis 20 Gew.-%, besonders bevorzugt 0,5 bis 15 Gew.-%, insbesondere bevorzugt 1 bis 10 Gew.-% bezogen auf die fertige Zubereitung.

Der Gewichtsanteil der eingesetzten Polymer-Mischung enthaltend ein oder mehrere Polymere der Komponente I) und ein oder mehrere Polymere der Komponente II) in den Emulsionen beträgt vorzugsweise 0,05 bis 10 Gew.-%, besonders bevorzugt 0,1 bis 5 Gew.% und insbesondere bevorzugt 0,2 bis 3 Gew.-%, bezogen auf die fertigen Zubereitungen.

Für die erfindungsgemäßen Zubereitungen auf wässrig-alkoholischer oder alkoholischer Basis kommen alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol oder Glycerin sowie Alkylenglykole, insbesondere Propylen-, Butylen- oder Hexylenglykol, und Mischungen aus den genannten Alkoholen. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und von Polyethylenglykol mit einer relativen Molekülmasse zwischen 400 und 600 bevorzugt.

Der Gewichtsanteil der Polymer-Mischung enthaltend ein oder mehrere Polymere der Komponente I) und ein oder mehrere Polymere der Komponente II) in wässrigen Zubereitungen oder in Zubereitungen auf wässrig-alkoholischer Basis beträgt 0,05 bis 10 Gew.-%, bevorzugt 0,1 bis 5 Gew.-%, besonders bevorzugt 0,2 bis 3 Gew.-%, bezogen auf die fertigen Zubereitungen.

Weitere bevorzugte Ausführungsformen sind Zubereitungen auf wässrig-tensidischer Basis.

Bevorzugt sind anionische, nichtionische und amphotäre Tenside.
Als anionische waschaktive Substanzen seien vorzugsweise genannt: C₁₀-C₂₀-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfat, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Amphoacetate- oder -glycinate, Acylglutamate, anionisch modifizierte Alkylpolyglukoside. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium sowie analogen Alkylammonium-Salze.

Der Gewichtsanteil der anionischen Tenside beträgt bevorzugt 1 bis 30 Gew.-%, besonders bevorzugt 5 bis 25 Gew.-%, insbesondere bevorzugt 10 bis 22 Gew.-%, bezogen auf die fertigen Zubereitungen.

Als nichtionische Tenside, die als waschaktive Substanzen eingesetzt werden können, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkylolamide, (Fettsäureamidpolyethylenglykole); N-Alkyl-, N-Alkoxypolyhydroxyfettsäureamid, Saccharoseester; Alkylpolyglukoside (APG^{®});Sorbitester und der Polyglykolether.

Der Gewichtsanteil der nichtionischen Tenside in den erfindungsgemäßen Zubereitungen liegt bevorzugt im Bereich von 1 bis 20 Gew.-%, besonders bevorzugt 2 bis 10 Gew.-%, insbesondere bevorzugt 3 bis 7 Gew.-%.

Bevorzugte Amphotenside sind: N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; N-Acylaminoalkyl-N,N-dimethyl-acetobetain, vorzugsweise N-(C₈-C₁₈)-Acyl-aminopropyl-N,N-dimethylacetobetain; (C₁₂-C₁₈)-Alkyl-dimethyl-sulfopropyl-betain; Amphotenside auf Basis Imidazolin (Handelsname: Miranol^{®}, Steinapon^{®}), vorzugsweise das Natrium-Salz des 1-(β-Carboxy-methyloxyethyl)-1-(carboxymethyl)-2-lauryl-imidazoliniums; Aminoxid, z.B. (C₁₂-C₁₈)-Alkyl-dimethylaminoxid, Fettsäureamidoalkyl-dimethylaminoxid.

Der Gewichtsanteil der amphotären Tenside in den erfindungsgemäßen Zubereitungen liegt bevorzugt im Bereich von 0,5 bis 20 Gew.-%, besonders bevorzugt von 1 bis 10 Gew.-%. Des Weiteren können in den erfindungsgemäßen Zubereitungen schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen Zubereitungen sind Alkylbetaine, insbesondere Cocoamidopropylbetain, Amphoacetate, Acylglutamate, insbesondere Natriumcocoylglutamat, Alkylethersulfosuccinate, insbesondere Di-natriumlaurethsulfosuccinat, Cocosfettsäurediethanolamid, Natriumcocoylisethionat, Natriummethylcocoyltaurat und Natriummethyllauroyltaurat.

Die Gesamtmenge der in den erfindungsgemäßen Zubereitungen eingesetzten Tenside beträgt vorzugsweise 1 bis 70 Gew.-%, besonders bevorzugt 5 bis 40 Gew.-%, insbesondere bevorzugt 12 bis 35 Gew.-%, bezogen auf die fertige Zubereitung.

Der Gewichtsanteil der eingesetzten Polymer-Mischung enthaltend ein oder mehrere Polymere der Komponente I) und ein oder mehrere Polymere der Komponente II) in wässrigen-tensidischen Zubereitungen beträgt 0,05 bis 10 Gew.-%, bevorzugt 0,1 bis 5 Gew.%, besonders bevorzugt 0,2 bis 3 Gew.-%, bezogen auf die fertigen Zubereitungen.

Die erfindungsgemäßen kosmetischen, pharmazeutischen und dermatologischen Zubereitungen können können als weitere Hilfs- und Zusatzstoffe Gelierungsmittel, Überfettungsmittel, feuchtigkeitsspendende Mittel, Silikone, Stabilisatoren, Konditioniermittel, Glycerin, Konservierungsmittel, Farbstoffe, Duft- und Parfümöle, Lösungsmittel, Hydrotrope, Trübungsmittel, Fettalkohole, Antischuppenmittel, Vitamine, Bisabolol, Allantoin, Phytantriol, Panthenol, AHA-Säuren, Pflanzenextrakte, beispielsweise Aloe Vera, Selbstbräunungsmittel, beispielsweise Dihydroxyaceton oder Erythrulose und Proteine enthalten.

Die gewünschte Viskosität der Zubereitungen kann durch Zugabe weiterer Verdickungsmittel eingestellt werden. In Betracht kommen vorzugsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Xanthan, Guar und Guarderivate, Scleroglucan, Traganth oder Dextrinderivate, insbesondere Dextrinester.

Als synthetische Polymere kommen verschiedene Materialien zum Einsatz, vorzugsweise Polyvinylalkohole, Polyacrylamide, Polyvinylamide, Polysulfonsäuren, insbesondere Copolymerisate auf Basis von Ammoniumsalzen von Acrylamidoalkylsulfonsäuren und cyclischen N-Vinylcarbonsäureamiden bzw. cyclischen und linearen N-Vinylcarbonsäureamiden oder auch hydrophob modifizierte Acrylamido-alkylsulfonsäure-Copolymerisate, Polyacrylsäure, Polyacrylsäurederivate, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den obengenannten Verbindungen, einschließlich ihrer verschiedenen Salze und Ester. Diese Polymere können wahlweise vernetzt oder unvernetzt sein.

Als Gelierungsmittel eignen sich alle oberflächenaktiven Stoffe, die in der flüssigen Phase gelöst eine Netzwerkstruktur ausbilden und so die flüssige Phase verfestigen. Geeignete Geliermittel sind z.B. in WO 98/58625 genannt.

Bevorzugte Gelierungsmittel sind Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher.

Bevorzugt enthalten die erfindungsgemäßen Zubereitungen 0,01 bis 20 Gew.-%, besonders bevorzugt 0,1 bis 10 Gew.-%, insbesondere bevorzugt 1 bis 8 Gew.-% und ganz besonders bevorzugt 3 bis 7 Gew.-% an Geliermitteln.

Weitere Zusatzstoffe können Siliconverbindungen sein, vorzugsweise Dimethylpolysiloxane, Methyl-phenylpolysiloxane, cyclische Silicone sowie amino-, fettsäure-, alkohol-, polyether-, epoyx-, fluor- und/oder alkylmodifizierte Siliconverbindungen, beispielsweise Phenyltrimethicone der Clariant GmbH wie SilCare^{®} 15M30, SilCare^{®} 15M40, SilCare^{®} 15M50, SilCare^{®} 15M60, Caprilyltrimethicone wie SilCare^{®} 31 M30, SilCare^{®} 31 M40, SilCare^{®} 31 M 50, SilCare^{®} 31 M 60, Alkylmethicone wie SilCare^{®} Silicone 41M10, SilCare^{®} Silicone 41 M15, SilCare^{®} Silicone 41 M20, SilCare^{®} Silicone 41 M30, SilCare^{®} 41 M40, SilCare^{®} 41 M50, SilCare^{®} 41 M65, SilCare^{®} 41 M70 oder SilCare^{®} 41M80, SilCare^{®} 41 M90,Trimethylsilyltrimethylsiloxylactat, Trimethylsilyltrimethylsiloxyglycolat, Trimethylsilyltrimethylsiloxysalicylat, Retinoxytrimethylsilan, Polyalkylarylsiloxane und Polyethersiloxan-Copolymere und modifizierte Polyorganosiloxane beispielsweise SilCare^{®} Silicone SEA (Clariant GmbH).

Die erfindungsgemäßen Zubereitungen können die oben genannten Siliconverbindungen vorzugsweise in den Gewichtsmengen von 0,1 bis 20 Gew.-%, besonders bevorzugt von 0,2 bis 15 Gew.-% und insbesondere bevorzugt 0,5 bis 10 Gew.-% bezogen auf die fertigen Zubereitungen enthalten.

Als Trägermaterialien in Betracht kommen vorzugsweise pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und Cellulose-Derivate.

Als fungizide Wirkstoffe können vorzugsweise Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrithion und Octopirox^{®} in den Gewichtsmengen von 0,05 bis 5 Gew.-%, bevorzugt 0,1 bis 3 Gew.-%, besonders bevorzugt 0,2 bis 2 Gew.-%, bezogen auf die fertigen Zubereitungen eingesetzt werden.
Die erfindungsgemäßen Zubereitungen können organische Säuren, bevorzugt α-oder β-Hydroxysäuren, besonders bevorzugt α-Hydroxysäuren enthalten.

Insbesondere bevorzugt sind α-Hydroxysäuren der Formel (6), 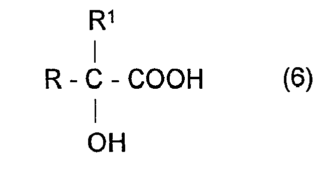 wobei R und R¹ unabhängig voneinander für H, F, Cl, Br, Alkyl-, Aralkyl- oder Arylgruppen mit gesättigten oder ungesättigten, linearen oder verzweigten Ketten, cyclischen Gruppen oder OH, CHO, COOH oder Alkoxygruppen mit 1 bis 9 Kohlenstoffatomen stehen können. Es kann die Säure und/oder das entsprechende Salz mit Alkali- oder Ammoniumgegenionen eingesetzt werden.

An sauren Komponenten in Betracht kommen Glykolsäure, Milchsäure, Methyllactonsäure, 2-Hydroxybutansäure, -pentan-, -hexan-, -heptan-, -octan-, -nonan-, -decan-, -undecan-, -dodecansäure (Laurylsäure), α-Hydroxymyristylsäure, α-Hydroxypalmitylsäure, α-Hydroxystearylsäure, Arachidonsäure, 2-Phenyl-2-hydroxyethansäure, Zitronensäure, Weinsäure, Mandelsäure, Salicylsäure, Ascorbinsäure, Brenztraubensäure, 2,2-Diphenyl-2-hydroxyethansäure, 3-Phenyl-2-Hydroxypropansäure, 2-Phenyl-2-Methyl-2-Hydroxyethansäure, 2-(4-Hydroxyphenyl)-2-Hydroxyethansäure, 2-(4'-Dichlorophenyl)-2-Hydroxyethansäure, 2-(3'-Hydroxy-4'-Methoxyphenyl)-2-Hydroxyethansäure, 2-(4'-Hydroxy-3'-Methoxyphenyl)-2-Hydroxyethansäure, 3-(2'-Hydroxyphenyl)-2-Hydroxypropansäure, 3-(4'-Hydroxyphenyl)-2-Hydroxypropansäure, 2-(3', 4'-Dihydroxyphenyl)-2-Hydroxyethansäure, Fumarsäure, Retinoesäure, aliphatische und organische Sulfonsäuren, Benzoesäure, Kojisäure, Fruchtsäure, Äpfelsäure, Gluconsäure, Galacturonsäure, saure Pflanzen- und/oder Fruchtextrakte und deren Derivate.

In einer weiteren bevorzugten Ausführungsform enthalten die Zubereitungen, bezogen auf das Gesamtgewicht der Zubereitungen, 0,01 bis 20 Gew.-%, vorzugsweise 0,5 bis 10 Gew.-% und besonders bevorzugt 1 bis 5 Gew.-% an Hydroxysäuren, vorzugsweise α-Hydroxy-säuren, wobei diese auch teilweise als Salz vorliegen können. In einer besonders bevorzugten Ausführungsform enthalten diese Zubereitungen, bezogen auf das Gesamtgewicht der Zubereitungen, 0,01 bis 10 Gew.-% an Polymer-Mischung enthaltend ein oder mehrere Polymere der Komponente I) und ein oder mehrere Polymere der Komponente II).

Insbesondere bevorzugt enthalten die erfindungsgemäßen Zubereitungen Glykolsäure, Milchsäure und/oder 2-Hydroxyoctansäure.

Die Hydroxysäure und das entsprechende Salz liegen vorzugsweise in einem molaren Verhältnis im Bereich von 1000 zu 1 bis 1 zu 1000, besonders bevorzugt 50 zu 1 bis 1 zu 50 vor.

Die erfindungsgemäßen Zubereitungen können vorteilhaft mit konventionellen Ceramiden, Pseudoceramiden, Fettsäure-N-alkylpolyhydroxyalkylamiden, Cholesterin, Cholesterinfettsäureestern, Fettsäuren, Triglyceriden, Cerebrosiden, Phospholipiden, Stoffen mit keratolytischer und keratoplastischer Wirkung und ähnlichen Stoffen, abgemischt werden.

Als feuchtigkeitsspendende Substanz stehen vorzugsweise Isopropylpalmitat, Glycerin und/oder Sorbitol zur Verfügung, die bevorzugt in den Gewichtsmengen 0,1 bis 50 % eingesetzt werden.

Als Selbstbräunungsmittel können vorzugsweise Dihydroxyaceton (DHA) und Erythrulose in den Gewichtsmengen 0,1 bis 10 %, bevorzugt 0,2 bis 8% verwendet werden.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide verwendet werden.

Als Konservierungsmittel in Betracht kommen vorzugsweise Phenoxyethanol, Parabene, insbesondere Butylparaben, Isobutylparabene Ethylparaben, Methylparaben, Propylparaben Pentandiol lmidazolidinylharnstoff oder Sorbinsäure.

### Bevorzugt eingesetzt werden nachfolgende Handelsprodukte:

Nipabutyl (Butylparaben), Nipagin (Ethylparaben), Nipagin M (Methylparaben), Nipasol M (Propylparaben), Nipabutyl Natriumsalz (Butylparaben, Natriumsalz), Nipagin A Natriumsalz (Ethylparaben, Natriumsalz), Nipagin M Natriumsalz (Methylparaben, Natriumsalz), Nipa Biopure 100 (Imidazolidinyl Harnstoff), Nipa Biopure 200 (Diazolidinyl Harnstoff), Nipaguard BNPD (2-Bromo-2-Nitropropane-1,3-diol), Nipaguard BNPD (2-Bromo-2-Nitropropane-1,3-diol), Nipaguard DMDMH (DMDM Hydantoin), Nipaguard SMG (Hydroxymethylglycinat, Natriumsalz), Phenoxetol (Phenoxyethanol), Propylene Phenoxetol (Phenoxyisopropanol), Nipasept (Methylparaben, Ethylparaben, Propylparaben), Nipastat (Methylparaben, Butylparaben, Ethylparaben, Propylparaben, lsobutylparaben), Nipasept Sodium (Methylparaben, Natriumsalz, Ethylparaben, Natriumsalz, Propylparaben, Natriumsalz), Nipastat Sodium (Butylparaben, Natriumsalz), Nipacide A (Methylparaben, Butylparaben, Isobutylparaben), Nipacide A Sodium (Methylparaben, Natriumsalz, Butylparaben, Natriumsalz, Isobutylparaben, Natriumsalz), Nipacombin A (Sodium Propylparaben, Sodium Methylparaben, Sodium Ethylparaben, Sodium Benzoates, Nipacombin SK (Sodium Propylparaben, Sodium Butylparaben, Nipaguard BPX (Phenoxyethanol, Methylparaben, Propylparaben, 2-Bromo-2-Nitropropane-1,3-diol), Nipaguard CMB (Triethylene Glycol, Benzyl Alcohol, Propylene Glycol, Methylchloroisothiazolinone and Methylisothiazolinone 3:1), Nipaguard DCB (Phenoxyethanol, Methyldibromo Glutaronitrile), Nipaguard IPF (PEG- 4 Laurate, Iodopropynyl Butylcarbamate), Nipaguard IPP2 (Phenoxyethanol, lodopropynyl Butylcarbamate), Nipaguard MPA (Benzyl Alcohol, Methylparaben, Propylparaben), Nipaguard MPS (Methylparaben, Propylparaben, Propylene Glycol), Nipaguard PBI (Iodopropynyl Butylcarbamate, Phenoxyethanol, Bronopol), Nipaguard PDU (Propylene Glycol, Diazolidinyl Urea, Methylparaben, Propylparaben), Nipaguard TBK (Phenoxyethanol, Methyldibromo Glutaronitrile, 2-Bromo-2-Nitropropane-1,3-diol, Butylparaben, Isobutylparaben), JM ActiCare (Silver Chloride, Titanium Dioxide, Diethylhexyl Sodium Sulfosuccinate, Propylene Glycol), Phenonip (Phenoxyethanol, Methylparaben, Ethylparaben, Butylparaben, Propylparaben, Isobutyiparaben), Phenosept (Chloroxylenol, Phenoxyisopropanol), Phenosept PG (Chloroxylenol, Phenoxyisopropanol, Propylene Glycol).
Sie werden vorzugsweise in den Gewichtsmengen von 0,001 bis 5 Gew.-%, besonders bevorzugt von 0,01 bis 3 Gew.-%, insbesondere bevorzugt von 0,1 bis 2 Gew.-%, bezogen auf die fertigen Zubereitungen eingesetzt.

Als Farbstoffe können die für kosmetische und pharmazeutische Zwecke geeigneten und zugelassenen Substanzen verwendet werden.

Als Duft- bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. die lonone, alphaIsomethylionon und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als Säuren oder Laugen zur pH-Wert Einstellung werden vorzugsweise Mineralsäuren, beispielsweise HCl, anorganische Basen, beispielsweise NaOH, KOH und organische Säuren, bevorzugt Zitronensäure verwendet.

Die Zubereitungen sind vorzugsweise auf einen pH Wert im Bereich 2 bis 10, bevorzugt pH 3 bis 8, besonders bevorzugt 4 bis 7 eingestellt.

In einer weiteren bevorzugten Ausführungsform der Erfindung sind die Zubereitungen klar transparent oder durchscheinend.

In einer weiteren bevorzugten Ausführungsform der Erfindung sind die Zubereitungen emulgator- und/oder ölfrei.

In einer weiteren bevorzugten Ausführungsform der Erfindung sind die Zubereitungen nicht klebrig.

In einer weiteren bevorzugten Ausführungsform der Erfindung stellen die Zubereitungen Gele dar und haben auf der Haut oder dem Haar eine lang anhaltende Geltextur.

In einer weiteren bevorzugten Ausführungsform der Erfindung vermitteln die Zubereitungen eine reichhaltige Sensorik.

Die erfindungsgemäßen Zubereitungen vermitteln ein angenehmes Gefühl auf Haut und Haar. Sie zeichnen sich zudem durch eine verbesserte Stabilität, insbesondere gegenüber Elektrolyten, aus. Weiterhin weisen sie auch ein sehr ästhetisches Erscheinungsbild auf.

In einer weiteren bevorzugten Ausführungsform der Erfindung enthalten die Zubereitungen als Komponente II) eine oder mehrere wasserlösliche oder wasserquellbare vernetzte copolymere Verdicker enthaltend ein oder mehrere Struktureinheiten der Formel (1), ein oder mehrere Struktureinheiten der Formel (2) und ein oder mehrere vernetzende Struktureinheiten, die aus Monomeren mit mindestens zwei olefinischen Doppelbindungen hervorgehen.

Die nachfolgenden Beispiele und Anwendungen sollen die Erfindung näher erläutern, ohne sie jedoch darauf zu beschränken (bei allen Prozentangaben handelt es sich um Gewichtsprozent).
Beispiele und Anwendungen

### 1) Darstellung der Makromonomeren

### Variante 1: Methacrylsäureglyzidylester

In einem ein Liter Dreihalskolben mit Rührer, Innenthermometer und Rückflusskühler werden 600 g Genapol^{®} T-250 vorgelegt und mit 75 g Methacrylsäureglyzidylester versetzt. Anschließend wird die Reaktionsmischung 2 Stunden lang auf 100°C erhitzt und der überschüssige Methacrylsäureglyzidylester im Vakuum abdestilliert. Das entstandene Makromonomere kann ohne weitere Reinigung in die Polymerisation eingesetzt werden.

### Variante 2: freie Meth-/Acrylsäure

In einem ein Liter Dreihaiskolben mit Rührer, Innenthermometer und Rückflusskühler werden 500 g Genapol^{®} UD-070 vorgelegt und mit 100 g Meth-/Acrylsäure und p-Toluolsulfonsäure als Katalysator versetzt. Anschließend wird die Reaktionsmischung 2 Stunden unter Rückfluss gekocht und die überschüssige Säure und das entstehende Reaktionswasser im Vakuum abdestilliert. Das entstandene Makromonomere kann ohne weitere Reinigung in die Polymerisation eingesetzt werden.

### Variante 3: Halogenderivate der Meth-/Acrylsäure

In einem ein Liter Dreihalskolben mit Rührer, Innenthermometer und Rückflusskühler werden 500 g Genapol^{®} UD-80 mit einer primären Aminoendgruppe vorgelegt und mit 110 g Meth-/Acrylsäurechlorid und 50g Natriumcarbonat versetzt. Anschließend wird die Reaktionsmischung 2 Stunden unter Rückfluss gekocht. Das Aufhören der CO₂-Entwicklung indiziert das Ende der Modifizierungsreaktion. Das überschüssige Säurechlorid wird im Vakuum abdestilliert. Das entstandene Makromonomere mit einer Meth-/Acrylamidendgruppe kann ohne weitere Reinigung in die Polymerisation eingesetzt werden.

### Variante 4: Ester der Meth-/Acrylsäure

In einem ein Liter Dreihalskolben mit Rührer, Innenthermometer und Rückflusskühler werden 500 g Genapol^{®} LA-070 vorgelegt und mit 100 g Meth-/Acrylsäuremethylester und 20 g Titantetraisopropylat versetzt. Anschließend wird die Reaktionsmischung 2 Stunden unter Rückfluss gekocht. Nachdem der entstehende Alkohol abdestilliert wurde wird der verbliebene Ester im Vakuum abdestilliert. Das entstandene Makromonomere mit einer Meth-/Acrylsäureendgruppe kann ohne weitere Reinigung in die Polymerisation eingesetzt werden.

### 2) Polymerisation

Allgemeine Polymerisationsvorschrift zur Herstellung der in den erfindungsgemäßen Zubereitungen eingesetzten Seitenkettenpolymere nach dem Fällungsverfahren in tert.-Butanol.

In einem 2-Liter Quickfitkolben mir Rückflusskühler, Gaseinleitung, Innenthermometer und Rührer werden 500 ml tert.-Butanol vorgelegt und mit der berechneten Menge an Acryloyldimethyltaurinsäure versetzt. Anschließend wird durch NH₃-Einleitung neutralisiert und die wie unter 1) hergestellten LCST (Lower critical solution temperature)-Seitenarme, d.h. die entsprechenden Makromonomere (auch mehrere unterschiedliche Spezies möglich), in die Reaktionsmischung hinzugegeben. Sollten weitere Comonomere benötigt werden, dann können diese nach der Neutralisation in die Reaktionsmischung hinzugegeben werden. Nach Inertisierung der Mischung mit N₂ oder Argon wird bei einer Innentemperatur von 60°C AIBN (Azobisisobutyronitril) als Initiator zugesetzt und die Polymerisationsreaktion eingeleitet. Nach wenigen Minuten kommt es zum Ausfällen des fertigen Polymeren. Die Mischung wird zwei Stunden auf Rückfluss erhitzt und das Polymerisat anschließend über eine Nutsche vom Lösungsmittel befreit und im Vakuum getrocknet. Diese Vorschrift ist allgemein auf alle im Weiteren beschriebenen Polymerisationsreaktionen anwendbar.

### Unvernetzte Seitenkettenpolymere (Polymere I)

### Beispiel 1: Reaktion gemäß allgemeiner Polymerisationsvorschrift

| Reaktand | Menge (g) |
|---|---|
| Makromonomer gemäß Variante 1 - Typ Genapol^{®}T-250 | 20 |
| NH₃-neutralisierte Acryloyldimethyltaurinsäure | 100 |
| tert.-Butanol | 300 |
| AIBN (Initiator) | 1 |

### Beispiel 2: Reaktion gemäß allgemeiner Polymerisationsvorschrift

| Reaktand | Menge (g) |
|---|---|
| Makromonomer gemäß Variante 3 - Typ Genapol^{®} UD-80 | 15 |
| Makromonomer gemäß Variante 1 - Typ Genapol^{®}T-250 | 15 |
| NH₃-neutralisierte Acryloyldimethyltaurinsäure | 90 |
| tert.-Butanol | 300 |
| AIBN (Initiator) | 1 |

### Beispiel 3: Reaktion gemäß allgemeiner Polymerisationsvorschrift

| Reaktand | Menge (g) |
|---|---|
| Makromonomer gemäß Variante 4 - Typ Genapol^{®} LA-070 | 18 |
| NH₃-neutralisierte Acryloyldimethyltaurinsäure | 80 |
| tert.-Butanol | 300 |
| Dilauroylperoxid (DLP) (Initiator) | 2 |

### Beispiel 4: Reaktion gemäß allgemeiner Polymerisationsvorschrift

Das Makromonomer aus Beispiel 4 wird analog Variante 1 hergestellt mit dem Unterschied, dass anstelle von 600 g Genapol^{®} T-250 600 g Genapol^{®} LA-070 verwendet werden.

| Reaktand | Menge (g) |
|---|---|
| Makromonomer gemäß Variante 1 - Typ Genapol^{®}LA-070 | 20 |
| Na-neutralisierte Acryloyldimethyltaurinsäure | 75 |
| Acrylamid | 50 |
| tert.-Butanol | 300 |
| AIBN (Initiator) | 1 |

### Beispiel 5: Reaktion gemäß allgemeiner Polymerisationsvorschrift

Das Makromonomer aus Beispiel 5 wird analog Variante 4 hergestellt mit dem Unterschied, dass anstelle von 500 g Genapol^{®} LA-070 500 g Genapol^{®} T-080 verwendet werden.

| Reaktand | Menge (g) |
|---|---|
| Makromonomer gemäß Variante 4 - Typ Genapol^{®}T-080 | 18 |
| NH₃-neutralisierte Acryloyldimethyltaurinsäure | 80 |
| tert.-Butanol | 300 |
| DLP (Initiator) | 2 |

### Anwendungs- und Formulierungsbeispiele

wässrige Refreshing-Gele, Beispiele 6 bis 28
Allgemeine Herstellweise für Refreshing-Gele

### Refreshing-Gel

Zusammensetzung: Beispiel 7

| | | |
|---|---|---|
| A | Carbopol^{®} 980 | 0,50 % |
| | Unvernetztes Copolymer mit Laureth-7 MA gemäß Beispiel 3 | 0,50 % |
| | Wasser | ad 100 |
| | NIPA^{®} BIOPURE 100 | 0,30 % |
| | NaOH (10 % in Wasser) | 1,60% |

### Herstellung

- I: Lösen der Komponenten A

Die Formulierungen 6, 8 bis 28 wurden analog der allgemeinen Herstellweise für Refreshing-Gele zubereitet (s. Tabellen 2a und 2b).

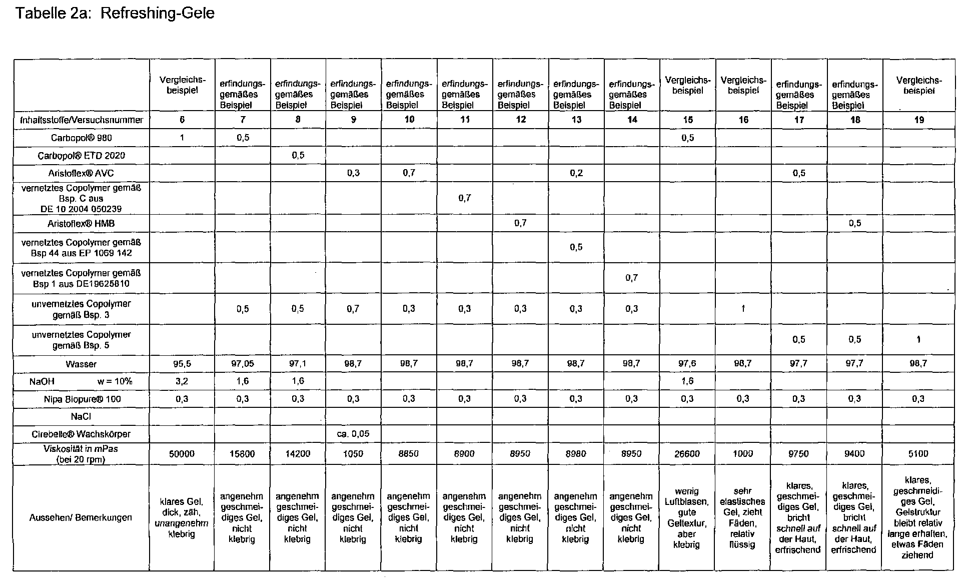

**Tabelle 2b: Fortsetzung Refreshing-Gele**

| | erfindungs-gemäßes Beispiel | erfindungs-gemäßes Beispiel | Vergleichs-beispiel | erfindungs-gemäßes Beispiel | Vergleichs-beispiel | Vergleichs-beispiel | Vergleichs-beispiel | erfindungs-gemäßes Beispiel | erfindungs-gemäßes Beispiel |
|---|---|---|---|---|---|---|---|---|---|
| Inhaltsstoffe/Versuchsnummer | 20 | 21 | 22 | 23 | 24 | 25 | 26 | 27 | 28 |
| Carbopol^{®} 980 | | | | | | | 1 | 0,5 | |
| Carbopol^{®} ETD 2020 | | | | | | | | | |
| Aristoflex^{®} AVC | 0,5 | 0,5 | | | | | | | |
| Aristoflex^{®} HMB | | | | | 1 | | | | 0,5 |
| unvernetztes Copolymer gemäß Bsp. 3 | 2 | 2 | | 0,5 | | | | 0,5 | 0,5 |
| unvernetztes Copolymer gemäß Bsp. 5 | | | | | | | | | |
| Wasser | ad 100 | ad 100 | ad 100 | ad 100 | ad 100 | ad 100 | ad 100 | ad 100 | ad 100 |
| NaOH w=10% | | | | | | | 3,2 | 1,6 | |
| Nipa Biopure^{®} 100 | | | 0,3 | 0,3 | 0,3 | 0,3 | 0,3 | 0,3 | 0,3 |
| NaCl | | | | | 0,1 | 0,1 | 0,1 | 0,1 | 0,1 |
| Propylenglykol | 2 | 2 | | | | | | | |
| Laureth-23 | 2 | 1,8 | | | | | | | |
| Fragrance | 0,2 | | | | | | | | |
| EtOH | | 20 | | | | | | | |
| Nipaguard^{®} MPA | 0,5 | | | | | | | | |
| Tylose^{®} H 10000 G4 | | | 1 | 0,5 | | 1 | | | |
| Cirebelle^{®} Wachskörper | | | | | | | | | |
| Viskosität in mPas (bei 20rpm) | 7500 | 1230 | 700 | 350 | 4800 | 760 | 39400 | 12200 | 1100 |
| Aussehen/ Bemerkungen | elastisches Gel, angenehme Geltextur auf der Haut | geschmeidiges Gel, weniger elastisch als **20** | relativ flüssig, geschmeidiges Gefühl leicht klebrig | relativ flüssig, angenehme Geltextur bleibt lange erhalten, bricht etwas schneller auf der Haut als **25** | klares Gel, viele Luftblasen, bricht sehr schnell auf der Haut | relativ flüssig, geschmeidiges Gefühl, leicht klebrig | geschmeidiges Gel, im Vergleich zu **27** klebrig | geschmeidiges Gel, viele Luftblasen, klar | einige Luftblasen bricht relativ schnell auf der Haut |

### Haar-Styling Gele, Beispiele 29 bis 51

Allgemeine Herstellweise für Haar-Styling Gele:
Haargel, sprühbar
Zusammensetzung: Beispiel 30

| | | |
|---|---|---|
| A | Aristoflex^{®} AVC | 0,15 % |
| | unvernetztes Copolymer mit Laureth-7 MA gemäß Beispiel 3 | 0,35 % |
| | Wasser | ad 100 |
| | NIPA^{®} BIOPURE 100 | 0,30 % |
| B | Diaformer^{®} Z-651 N | 4,00 % |

### Herstellung

- I: Lösen der Komponenten A
- II: Zugabe von B zu I.

Die Formulierungen 29, 31 bis 51 wurden analog der allgemeinen Herstellweise für Haar-Styling Gele zubereitet (s. Tabellen 3a und 3b).

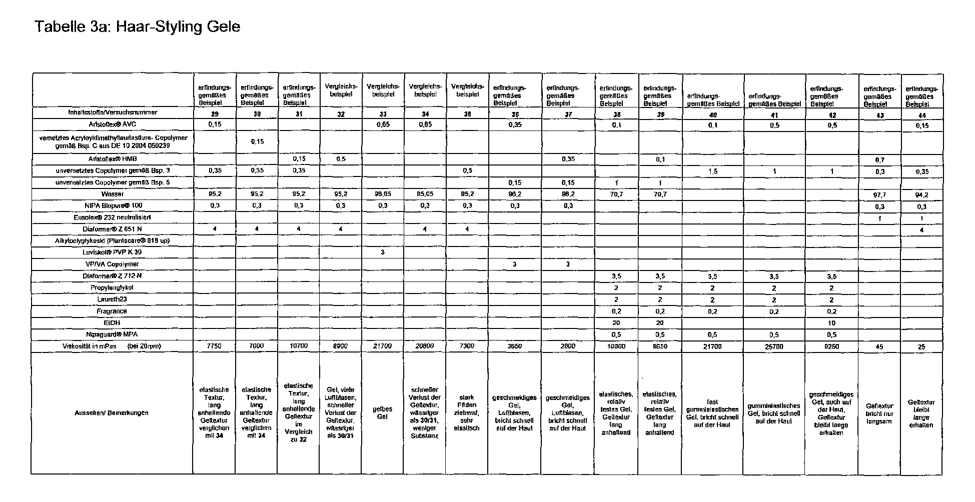

**Tabelle 3b: Fortsetzung Haar-Styling Gele**

| | Vergleichs-beispiel | erfindungs-gemäßes Beispiel | erfindungsgemäßes Beispiel | Vergleichs-beispiel | erfindungsgemäßes Beispiel | erfindungs-gemäßes Beispiel | erfindungs-gemäßes Beispiel |
|---|---|---|---|---|---|---|---|
| Inhaltsstoffe/Versuchs-nummer | **45** | **46** | **47** | **48** | **49** | **50** | **51** |
| Aristoflex^{®} AVC | | 0,5 | 0,1 | | 0,5 | 0,5 | 0,1 |
| unvernetztes Copolymer gemäß Bsp. 3 | 1,5 | 2 | 2 | 1,5 | 2 | 2 | 2 |
| Wasser | 95,2 | 70 | 69,9 | 94,7 | 71,92 | 65,9 | 60,8 |
| NIPA Biopure^{®} 100 | 0,3 | | | 0,3 | | | |
| Diaformer^{®} Z 651 N | | | | | | | |
| VP/VA Copolymer | 3 | 3 | | 3 | 3 | 3 | |
| Diaformer^{®} Z 712 N | | | 3,5 | | | | 5 |
| Propylenglykol | | 2 | | | 2 | | |
| Laureth-23 | | 2 | 2 | | | 2 | 2 |
| Fragrance | | 0,2 | 0,2 | | | 0,2 | 0,2 |
| EtOH | | 20 | 20 | | 20 | 20 | 20 |
| Nipaguard^{®} MPA | | 0,5 | 0,5 | | 0,5 | 0,5 | 0,5 |
| Genapol ^{®}LA 070 | | | | | | 2 | 2 |
| Silcare^{®} Silicone SEA | | | | | | 1 | 1 |
| Pigment: Cloisonne Super Green^{®} | | | | | ~ 0,04 ~ | | |
| Glitter (Creasparkles Metallic Silver 700^{®}) | | | | | ~ 0,04 | | |
| Abil^{®} EM 90 (Silikonemulgator) | | | | | | 1 | 1 |
| Viskosität in mPas (bei 20rpm) | 370 | 2850 | 3840 | 11700 | 1390 | 9550 | 2120 |
| Aussehen/ Bemerkungen | dünnflüssiges Gel, zieht Fäden Gel, zieht | Gel, angenehm auf der Haut, Geltextur bleibt lange erhalten | festes, elastisches Gel, angenehm auf der Haut, Geltextur bleibt lange erhalten, Schimmereffekt gut erkennbar | Gel | dünnflüssiges Gel, angenehm auf der Haut, Geltextur bleibt lange erhalten, Glitter erkennbar | dünnflüssiges Gel, angenehm auf der Haut, Geltextur bleibt lange erhalten | elastisches Gel, angenehm auf der Haut, Geltextur bleibt lange erhalten, Schimmereffekt ist erkennbar |

### Milde Reinigungsgele, Beispiele 52 bis 55

Allgemeine Herstellweise für milde Reinigungsgele:
Mildes Reinigungsgel
Zusammensetzung: Beispiel 54

| | | |
|---|---|---|
| A | Aristoflex^{®} AVC | 0,30 % |
| | unvernetztes Copolymer mit Laureth-7 MA gemäß Beispiel 3 | 0,70 % |
| | Wasser | ad 100 % |
| | NIPA^{®} BIOPURE 100 | 0,30 % |
| B | Genapol^{®} LRO | 25,90 % |
| | Genagen^{®} CAB 818 | 10,00 % |

### Herstellung:

- I: Lösen der Komponenten A
- II: Mischen der Komponenten B
- III: Zugabe von II zu I

Die Formulierungen 52, 53 und 55 wurden analog der allgemeinen Herstellweise für milde Reinigungsgele zubereitet (s. Tabelle 4).

**Tabelle 4: milde Reinigungsgele**

| | erfindungs- gemäßes Beispiel | erfindungs-gemäßes Beispiel | erfindungs-gemäßes Beispiel | erfindungs-gemäßes Beispiel |
|---|---|---|---|---|
| Inhaltsstoffe/Versuchsnummer | **52** | **53** | **54** | **55** |
| Aristoflex® AVC | 0,3 | | 0,3 | |
| Aristoflex® HMB | | 0,3 | | 0,3 |
| unvernetztes Copolymer gemäß Bsp. 3 | 0,7 | 0,7 | 0,7 | 0,7 |
| Wasser | 92,7 | 92,7 | 62,8 | 62,8 |
| NIPA Biopure® 100 | 0,3 | 0,3 | 0,3 | 0,3 |
| Alkylpolyglykosid (Plantacare® 818 up) | 6 | 6 | | |
| Genapol® LRO | | | 25,9 | 25,9 |
| Genagen® CAB 818 | | | 10 | 10 |
| Viskosität in mPas (bei 20 rpm) | 2880 | 1615 | 250 | 1560 |
| Aussehen/ Bemerkungen | elastisch, gelartig | elastisch, gelartig | Gel | Gel |

### Sonnenschutzgele, Beispiele 56 bis 61

Allgemeine Herstellweise für Sonnenschutzgele:
Sonnenschutzgel
Zusammensetzung: Beispiel 60

| | | |
|---|---|---|
| A | Eusolex^{®} 232 | 1,00 % |
| | Water | ad 100 % |
| | NIPA^{®} BIOPURE 100 | 0,30 % |
| | | |
| B | Aristoflex^{®} AVC | 0,70 % |
| | Unvernetztes Copolymer mit Steareth-8 MA gemäß Beispiel 5 | 0,30 % |

### Herstellung

- I: Mischen der Komponenten A und Einstellen auf pH 7,3 mit Tris(hydroxmethyl)aminomethan
- II: Nacheinander Zugabe der Komponenten B zu I.

Die Formulierungen 56 bis 59 und 61 wurden analog der allgemeinen Herstellweise für Sonnenschutzgele zubereitet (s. Tabelle 5).

**Tabelle 5: Sonnenschutzgele**

| | Vergleichs-beispiel | erfindungs-gemäßes Beispiel | erfindungs-gemäßes Beispiel | erfindungs-gemäßes Beispiel | erfindungs-gemäßes Beispiel | erfindungs-gemäßes Beispiel |
|---|---|---|---|---|---|---|
| Inhaltsstoffe/Versuchsnummer | **56** | **57** | **58** | **59** | **60** | **61** |
| Aristoflex® AVC | | 0,3 | | 0,7 | 0,7 | |
| Aristoflex® HMB | | | 0,3 | | | 0,7 |
| unvernetztes Copolymer gemäß Bsp.3 | 1 | 0,7 | 0,7 | 0,3 | | |
| unvernetztes Copolymer gemäß Bsp.5 | | | | | 0,3 | 0,3 |
| Wasser | 97,7 | 97,7 | 97,7 | 97,7 | 97,7 | 97,7 |
| NIPA Biopure® 100 | 0,3 | 0,3 | 0,3 | 0,3 | 0,3 | 0,3 |
| Eusolex® 232 neutralisiert | 1 | 1 | 1 | 1 | 1 | 1 |
| Viskosität in mPas (bei 20 rpm) | 4350 | 2920 | 1610 | 945 | 460 | 60 |
| Aussehen / Bemerkungen | Fäden ziehend, klebt leicht im Trockenen | angenehmere Textur als **56** | angenehmere Textur als **56** | angenehm auf der Haut | angenehm auf der Haut | angenehm auf der Haut |

### Stylingcremes, Beispiele 62 bis 72

Allgemeine Herstellweise für Stylingcremes:
Stylingcreme
Zusammensetzung: Beispiel 62

| | | |
|---|---|---|
| A | Mineralöl, niedrigviskos | 1,00 % |
| | Cetiol^{®} 868 | 1,00 % |
| | SilCare^{®} 15M50 | 1,50 % |
| | Hostaphat^{®} KL 340 D | 1,00 % |
| | | |
| B | Aristoflex^{®} AVC | 1,05 % |
| | unvernetztes Copolymer mit Laureth-7 MA gemäß Beispiel 3 | 0,45 % |
| | | |
| C | Wasser | ad 100,00 % |
| | Glycerin | 3,00 % |
| | Phenonip^{®} | 0,40 % |
| | | |
| D | Diaformer^{®} Z 711 N | 3,00 % |

### Herstellung

- I: Mischen von A und B
- II: Einrühren von C in I
- III: Einrühren von D in II
- IV: Homogenisieren

Die Formulierungen 63 bis 72 wurden analog der allgemeinen Herstellweise für Stylingcremes zubereitet (s. Tabelle 6).

**Tabelle 6: Stylingcremes**

| | erfindungs-gemäßes Beispiel | erfindungs-gemäßes Beispiel | erfindungs-gemäßes Beispiel | erfindungs-gemäßes Beispiel | Vergleichs-beispiel | Vergleichs-beispiel | Vergleichs-beispiel | Vergleichs-beispiel | Vergleichs-beispiel | erfindungs-gemäßes Beispiel | Vergleichs-beispiel |
|---|---|---|---|---|---|---|---|---|---|---|---|
| Inhaltsstoffe/Versuchsnummer | 62 | 63 | 64 | 65 | 66 | 67 | 68 | 69 | 70 | 71 | 72 |
| Aristoflex®AVC | 1,05 | 0,45 | 1,05 | 1,05 | | | | | | 1,05 | |
| Aristoflex® HMB | | | | | | | | | | | |
| unvernetztes Copolymer gemäß Bsp. 3 | 0,45 | 1,05 | 0,45 | 0,45 | 1 | 1 | 2 | 1 | 1 | 1,05 | 1,5 |
| Wasser | 87,6 | 87,6 | 87,6 | 87,6 | 88,1 | 88,1 | 87,1 | 88,1 | 88,1 | 87,6 | 86,6 |
| VP/VA Copolymer | | | | | | 3 | 3 | | 3 | | 3 |
| Paraffinöl | 1 | 1 | 1 | 1 | 1 | 1 | 1 | 1 | 1 | 1 | 1 |
| Cetiol® 868 | 1 | 1 | 1 | 1 | 1 | 1 | 1 | 1 | 1 | 1 | 1 |
| Silcare® 15M50 | 1,5 | 1,5 | 1,5 | 1,5 | 1,5 | 1,5 | 1,5 | 1,5 | 1,5 | 1,5 | 1,5 |
| Tegin® M | | | 0,3 | 0,3 | 1 | 1 | 0,3 | | | | |
| Hostaphat® KL 340 D | 1 | 1 | 0,7 | | | | 0,7 | | | | |
| Phenonip® | 0,4 | 0,4 | 0,4 | 0,4 | 0,4 | 0,4 | 0,4 | 0,4 | 0,4 | 0,4 | 0,4 |
| Glycerin | 3 | 3 | 3 | 3 | 3 | 3 | 3 | 3 | 3 | 3 | 3 |
| Diaformer® Z 711 N | 3 | 3 | 3 | 3 | 3 | | | 3 | | 3 | 3 |
| Hostacerin® DGL | | | | 0,7 | | | | | | | |
| Hostacerin® DGI | | | | | | | | 1 | 1 | 1 | 2 |
| Aussehen/ Bemerkungen | Creme, gute Sensorik auf der Haut | angenehme Sensorik, sehr geschmeidig | Creme, auf der Haut angenehm | Creme, auf der Haut angenehm | keine homogene Creme, Feststoffab scheidung | keine homogene Creme, Feststoffab scheidung | elastische Creme | unangenehm elastisch, Fäden ziehend, sehr dünn | elastische Creme | sehr angenehme Creme, sehr gute Sensorik auf der Haut | elastische Creme |

### Hautcreme (O/W-Emulsion), Beispiele 73 bis 75

Allgemeine Herstellweise für O/W-Cremes:
O/W Creme
Zusammensetzung: Beispiel 74

| | | |
|---|---|---|
| A | Mineralöl, niedrigviskos | 1,00 % |
| | Cetiol^{®} 868 | 1 ,00 % |
| | SilCare^{®} 15M50 | 1,50 % |
| | Hostaphat^{®} KL 340 D | 1,00 % |
| | | |
| B | Aristoflex^{®} AVC | 1,05 % |
| | unvernetztes Copolymer mit | |
| | Laureth-7 MA gemäß Beispiel 3 | 0,45 % |
| | | |
| C | Wasser | ad 100,00 % |
| | Glycerin | 3,00 % |
| | Phenonip^{®} | 0,40 % |

### Herstellung

- I: Mischen von A und B
- II: Einrühren von C in I
- III: Homogenisieren

Die Formulierungen 73 und 75 wurden analog der allgemeinen Herstellweise für O/WCremes zubereitet (s. Tabelle 7).

**Tabelle 7: O/W Cremes**

| | Vergleichsbeispiel | erfindungsgemäßes Beispiel | Vergleichsbeispiel |
|---|---|---|---|
| Inhaltsstoffe/Versuchsnummer | 73 | 74 | 75 |
| Aristoflex^{®} AVC | | 1,05 | |
| unvernetztes Copolymer gemäß Beispiel 3 | 1 | 0,45 | 1,5 |
| Wasser | 88,1 | 87,6 | 86,6 |
| Paraffinöl | 1 | 1 | 1 |
| Cetiol^{®} 868 | 1 | 1 | 1 |
| Silcare Silicone^{®} 15M50 | 1,5 | 1,5 | 1,5 |
| Tegin^{®} M | 0,3 | | |
| Hostaphat^{®} KL 340 D | 0,7 | | |
| Phenonip^{®} | 0,4 | 0,4 | 0,4 |
| Glycerin | 3 | 3 | 3 |
| Hostacerin^{®} CGI | | | 2 |
| Aussehen / Bemerkungen | Produkt trennt über Nacht | Creme, angenehm auf der Haut, stabil | dünnflüssig, trennt über Nacht |

Anti-aging Gele, Beispiele 76 bis 79
Allgemeine Herstellweise für Anti-aging Gele:
Anti-aging Gel
Zusammensetzung: Beispiel 77

| | | |
|---|---|---|
| A | Aristoflex^{®} HMB | 0,30 % |
| | Unvernetztes Copolymer mit Laureth-7 MA gemäß Beispiel 3 | 0,70 % |
| | Wasser | ad 100 % |
| | NIPA^{®} BIOPURE 100 | 0,30 % |
| B | Glycolsäure, (30 % in Wasser)* * neutralisiert mit NaOH auf pH 4 | 3,33 % |

### Herstellung

- I: Lösen der Komponenten A
- II: Zugabe von B in I

Die Formulierungen 76, 78, 79 wurden analog der allgemeinen Herstellweise für anti-aging Gele zubereitet (s. Tabelle 8).

**Tabelle 8: anti-aging Gele**

| | erfindungsgemäßes Beispiel | erfindungsgemäßes Beispiel | erfindungsgemäßes Beispiel | erfindungsgemäßes Beispiel |
|---|---|---|---|---|
| Inhaltsstoffe/Versuchsnummer | 76 | 77 | 78 | 79 |
| Aristoflex^{®} AVC | 0,3 | | 0,3 | |
| Aristoflex^{®} HMB | | 0,3 | | 0,3 |
| unvernetztes Copolymer gemäß Beispiel 3 | 0,7 | 0,7 | | |
| unvernetztes Copolymer gemäß Beispiel 5 | | | 0,7 | 0,7 |
| Wasser | 99,7 | 99,7 | 95,4 | 95,4 |
| NIPA Biopure^{®} 100 | 0,3 | 0,3 | 0,3 | 0,3 |
| Glykolsäure, neutralisiert | 3,33 | 3,33 | 3,33 | 3,33 |
| Viskosität in mPas (bei 20 rpm) | 880 | 795 | 190 | 55 |
| Aussehen / Bemerkungen | stabiles Gel | stabiles Gel | Fluid | Fluid |

### Chemische Bezeichnung (INCI-Nomenklatur) der eingesetzten Handelsprodukte:

| | |
|---|---|
| Abil^{®} EM-90 | Cetyl PEG/PPG-10/1 Dimethicone |
| Aristoflex^{®} AVC | Ammonium Acryloyldimethyltaurat/VP Copolymer |
| Aristoflex^{®} HMB | Ammonium Acryloyldimethyltaurat/Beheneth-25 |
| Methacrylat | Crosspolymer |
| Carbopol^{®} 980 | Carbomer |
| Cetiol^{®} 868 | Ethylhexylstearat |
| Cloisonne^{®} Super Green | Mica and Titanium Dioxide and Iron Oxides and Ferric Ferrocyanide |
| Creasparkles Metallic | Polyethylene Terephthalate and Glycerin and |
| Silver 700^{®} (Glitter) | Acrylamide/Ammonium Acrylate Copolymer and Aluminum Powder |
| Diaformer^{®} Z-651 N | Acrylates/Lauryl Acrylate/Stearyl Acrylate/Ethylamine Oxide Methacrylate Copolymer |
| Diaformer^{®} Z 711 N | Acrylates/Lauryl Acrylate/Stearyl Acrylate/Ethylamine Oxide Methacrylate Copolymer |
| Diaformer^{®} Z 712 N | Acrylates/Lauryl Acrylate/Stearyl Acrylate/Ethylamine Oxide Methacrylate Copolymer |
| Eusolex^{®} 232 | Phenylbenzimidazole Sulfonic Acid |
| Genagen^{®} CAB 818 | Cocamidopropyl Betaine |
| Genapol^{®} LA 070 | Laureth-7 |
| Genapol^{®} LRO | Sodium Laureth Sulfate |
| Hostacerin^{®} DGI | Polyglyceryl-2 Sesquiisostearate |
| Hostacerin^{®} DGL | PEG-10 Polyglyceryl-2 Laurate |
| Hostaphat^{®} KL 340 D | Trilaureth- 4 Phosphat |
| NIPA Biopure^{®} 100 | Imidazolidinyl Urea (Imidazolidinylharnstoff) |
| Nipaguard^{®} MPA | Benzyl Alcohol and Methylparaben and Propylparaben |
| Phenonip^{®} | Phenoxyethanol and Methylparaben and Ethylparaben and Butylparaben and Propylparaben and Isobutylparaben |
| Plantacare^{®} 818 up | Coco-Glucoside |
| Luviskol^{®} PVP K 30 | PVP (Polyvinylpyrrolidone) |
| SilCare^{®}® Silicone SEA | Trideceth-9 PG-Amodimethicone and Trideceth-12 |
| SilCare^{®} 15M50 | Phenyltrimethicone |
| Tegin^{®} M | Glyceryl Stearate |
| Tylose^{®} H 10000 G4 | Hydroxyethylcellulose |

## Patentansprüche

1. Kosmetische oder pharmazeutische Zubereitung, enthaltend
I) ein oder mehrere wasserlösliche unvernetzte Copolymere, enthaltend
A) eine oder mehrere Struktureinheiten der Formel (1) 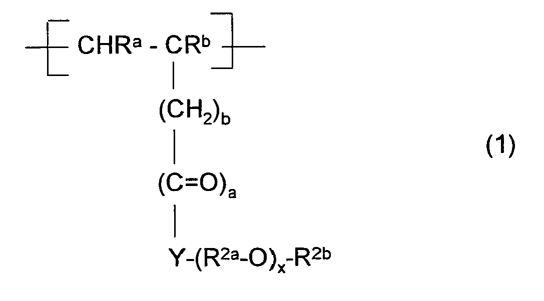 worin
R^{a} H oder CH₃;
R^{b} H oder CH₃;
a 0 oder 1;
b 0 oder 1;
Y O, S, PH oder NH;
R^{2a} eine lineare oder verweigte (C₂-C₄)-Alkylengruppe;
x eine ganze Zahl zwischen 1 und 500; und
R^{2b} Wasserstoff oder einen gesättigten oder ein- oder mehrfach ungesättigten linearen oder verzweigten aliphatischen, cycloaliphatischen oder aromatischen (C₁-C₃₀)-Kohlenwasserstoffrest bedeuten und
B) eine oder mehrere Struktureinheiten der Formel (2) 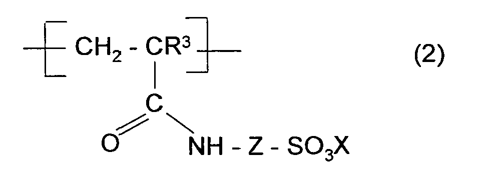
worin
R³ für Wasserstoff, Methyl oder Ethyl steht,
Z für (C₁-C₈)-Alkylen steht, und
X für Wasserstoff, Lithium, Natrium, Kalium, Magnesium, Calcium, Ammonium, Monoalkylammonium, Dialkylammonium, Trialkylammonium oder Tetraalkyl-ammonium, wobei die Alkylsubstituenten der Ammoniumionen unabhängig voneinander (C₁-C₂₂)-Alkylreste darstellen, die mit 0 bis 3 Hydroxyalkylgruppen besetzt sein können, deren Alkylkettenlänge in einem Bereich von C₂ bis C₁₀ variieren kann, steht oder X auch für ein bis dreifach ethoxylierte Ammoniumverbindungen mit gleichem oder unterschiedlichem Ethoxylierungsgrad steht, und wobei in den unvernetzten Copolymeren auch Struktureinheiten der Formel (2) mit unterschiedlichem X enthalten sein können,
und
II) ein oder mehrere wasserlösliche oder wasserquellbare vernetzte oder unvernetzte copolymere oder homopolymere Verdicker.

2. Zubereitung nach Anspruch 1, **dadurch gekennzeichnet, dass** R^{a}, R^{b}, a und b in der Struktureinheit der Formel (1) aus folgenden Kombinationen ausgewählt sind:
R^{a} = R^{b} = H und a = b = 0;
R^{a} = R^{b} = H, a = 0 und b = 1;
R^{a} = R^{b} = H, a = 1 und b = 0; oder
R^{a} = H, R^{b} = CH₃, a = 1 und b = 0.

3. Zubereitung nach Anspruch 2, **dadurch gekennzeichnet, dass** R^{a}, R^{b}, a und b in der Struktureinheit der Formel (1) aus folgenden Kombinationen ausgewählt sind:
R^{a} = R^{b} = H, a = 1 und b = 0 oder
R^{a} = H, R^{b} = CH₃, a = 1 und b = 0.

4. Zubereitung nach einem oder mehreren der Ansprüche 1 bis 3, **dadurch gekennzeichnet, dass** in der Struktureinheit der Formel (1)
R^{2a} für einen Ethylen- oder Propylenrest, vorzugsweise für einen Ethylenrest, steht,
x für eine Zahl zwischen 3 und 50, vorzugsweise zwischen 6 und 30, steht, und
R^{2b} für einen gesättigten oder einen ein- oder mehrfach ungesättigten aliphatischen oder cycloaliphatischen Kohlenwasserstoffrest steht.

5. Zubereitung nach einem oder mehreren der Ansprüche 1 bis 4, **dadurch gekennzeichnet, dass** R^{2b} in der Struktureinheit der Formel (1) für einen (C₆-C₂₂)-Kohlenwasserstoffrest, vorzugsweise für einen (C₁₂-C₁₈)-Kohlenwasserstoffrest, steht.

6. Zubereitung nach Anspruch 5, **dadurch gekennzeichnet, dass** der Kohlenwasserstoffrest ein Alkyl- oder ein ein- oder mehrfach ungesättigter Alkenylrest, vorzugsweise ein Alkylrest, ist.

7. Zubereitung nach einem oder mehreren der Ansprüche 1 bis 6, **dadurch gekennzeichnet, dass** R^{2b} in der Struktureinheit der Formel (1) für einen Rest ausgewählt aus Stearyl, Lauryl, Cocoyl, Undecyl, Behenyl, Cetearyl, Cetyl und Myristyl und vorzugsweise für einen Rest ausgewählt aus Stearyl, Lauryl, Cetyl und Myristyl steht.

8. Zubereitung nach einem oder mehreren der Ansprüche 1 bis 7, **dadurch gekennzeichnet, dass** in der Struktureinheit der Formel (2) R³ für H, Z für -C(CH₃)₂-CH₂- und X für Wasserstoff, Natrium, Kalium oder Ammonium, vorzugsweise für H oder Ammonium, steht und wobei in den unvernetzten Copolymeren auch Struktureinheiten der Formel (2) mit unterschiedlichem X enthalten sein können.

9. Zubereitung nach einem oder mehreren der Ansprüche 1 bis 8, **dadurch gekennzeichnet, dass** der Neutralisierungsgrad der Struktureinheit der Formel (2) 70 bis 100 mol-%, vorzugsweise 80 bis 100 mol-% und besonders bevorzugt 80 bis 99 mol-% beträgt.

10. Zubereitung nach einem oder mehreren der Ansprüche 1 bis 9, **dadurch gekennzeichnet, dass** die molaren Anteile der Struktureinheit der Formel (1) und der Struktureinheit der Formel (2) im Copolymer der Komponente I) jeweils von 0,1 bis 99,9 mol-% betragen.

11. Zubereitung nach einem oder mehreren der Ansprüche 1 bis 10, **dadurch gekennzeichnet, dass** der Anteil der Struktureinheit der Formel (1) im Copolymer der Komponente I) von 50,1 bis 99,9 mol-%, bevorzugt von 70 bis 95 mol-% und besonders bevorzugt von 80 bis 90 mol-% beträgt.

12. Zubereitung nach einem oder mehreren der Ansprüche 1 bis 10, **dadurch gekennzeichnet, dass** der Anteil der Struktureinheit der Formel (1) im Copolymer der Komponente I) von 0,1 bis 50 mol-%, bevorzugt von 5 bis 25 mol-% und besonders bevorzugt von 6 bis 15 mol-% beträgt.

13. Zubereitung nach einem oder mehreren der Ansprüche 1 bis 12, **dadurch gekennzeichnet, dass** der oder die wasserlöslichen oder wasserquellbaren vernetzten oder unvernetzten copolymeren oder homopolymeren Verdicker der Komponente II) ausgewählt sind aus
a) Polymeren auf Basis von Methacrylsäure oder Acrylsäure und modifizierter (Meth)acrylsäure, vorzugsweise vernetzten Polymeren der Acrylsäure, Copolymeren aus (Meth)acrylsäure und Polyalkylenpolyether, hydrophob modifizierten Poly(meth)acrylaten,
b) Homopolymeren der Dimethylaminoethyl(meth)acrylate, quaternisiert mit Methylchlorid,
c) Copolymeren von Dimethylaminoethyl(meth)acrylat, quaternisiert mit Methylchlorid und Acrylamid,
d) vernetzten Copolymeren von Vinylisodecanoat und (Meth)acrylsäure,
e) Polyvinylalkoholen,
f) Polyvinylmethylethern
g) Polyacrylamiden,
h) Polyvinylamiden,
i) Polyvinylpyrrolidon,
j) Poly(meth)acrylsäuren, Poly(meth)acrylsäureestern und weiteren Poly(meth)acrylsäurederivaten,
k) Polyethylenoxiden,
l) Copolymeren aus Maleinsäureanhydrid und Vinylmethylether,
m) Polysulfonsäuren, vorzugsweise Copolymerisaten auf Basis der Acrylamido-alkylsulfonsäure und/oder deren Salzen und einem oder mehreren Comonomeren ausgewählt aus cyclischen N-Vinylcarbonsäureamiden und linearen N-Vinylcarbonsäureamiden oder hydrophob modifizierten vernetzten Acrylamido-alkylsulfonsäure-Copolymerisaten
n) vernetzten Homopolymeren der Acrylamido-alkylsulfonsäure und/oder deren Salzen,
o) Copolymeren aus der Acrylamido-alkylsulfonsäure und/oder deren Salzen und Comonomeren ausgewählt aus Acrylamid, Hydroxyethyl(meth)acrylat und kationisch modifizierte (Meth)acrylate.
p) natürlichen und modifizierten natürlichen Polymeren auf Basis von Polysacchariden, vorzugsweise Celluloseether, Cellulosederivate, Carboxymethylcellulose, Hydroxyethylcellulose, Gelatine, Stärke und Stärkederivate, Natriumalginate, Xanthan, Guar und Guarderivate, Scleroglucan,Traganth oder Dextrinderivate, insbesondere Dextrinester.

14. Zubereitung nach einem oder mehreren der Ansprüche 1 bis 13, **dadurch gekennzeichnet, dass** der oder die wasserlöslichen oder wasserquellbaren vernetzten oder unvernetzten copolymeren oder homopolymeren Verdicker der Komponente II) ausgewählt sind aus
a) Polymeren auf Basis von Methacrylsäure oder Acrylsäure und modifizierter (Meth)acrylsäure, vorzugsweise vernetzten Polymeren der Acrylsäure, Copolymeren aus (Meth)acrylsäure und Polyalkylenpolyether, hydrophob modifizierten Poly(meth)acrylaten,
g) Polyacrylamiden,
j) Poly(meth)acrylsäuren, Poly(meth)acrylsäureestern und weiteren Poly(meth)acrylsäurederivaten,
m) Polysulfonsäuren, vorzugsweise Copolymerisaten auf Basis der Acrylamido-alkylsulfonsäure und/oder deren Salzen und einem oder mehreren Comonomeren ausgewählt aus cyclischen N-Vinylcarbonsäureamiden und linearen N-Vinylcarbonsäureamiden oder hydrophob modifizierten vernetzten Acrylamido-alkylsulfonsäure-Copolymerisaten,
n) Vernetzten Homopolymeren der Acrylamido-alkylsulfonsäure und/oder deren Salzen,
o) Copolymeren aus der Acrylamido-alkylsulfonsäure und/oder deren Salzen und Comonomeren ausgewählt aus Acrylamid, Hydroxyethyl(meth)acrylat und kationisch modifizierten (Meth)acrylaten und
p) natürlichen und modifizierten natürlichen Polymeren auf Basis von Polysacchariden, vorzugsweise Celluloseether, Cellulosederivate, Carboxymethylcellulose, Hydroxyethylcellulose, Gelatine, Stärke und Stärkederivate, Natriumalginate, Xanthan, Guar und Guarderivate, Scleroglucan,Traganth oder Dextrinderivate, insbesondere Dextrinester.

15. Zubereitung nach einem oder mehreren der Ansprüche 1 bis 14, **dadurch gekennzeichnet, dass** der Gewichtsanteil an vernetzenden Comonomeren, bezogen auf die Gesamtmasse der Polymere der Komponente II) von 0 bis 20 Gew.-% beträgt.

16. Zubereitung nach einem oder mehreren der Ansprüche 1 bis 15, **dadurch gekennzeichnet, dass** der oder die wasserlöslichen oder wasserquellbaren vernetzten oder unvernetzten copolymeren oder homopolymeren Verdicker der Komponente II) ausgewählt sind aus Copolymeren, bestehend im wesentlichen aus a1) 1 bis 50 Gew.-% der wiederkehrenden Struktureinheit der Formel (3) 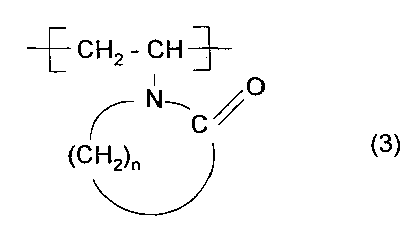 wobei n eine ganze Zahl von 2 bis 9 bedeutet
oder
a2) 1 bis 50 Gew.-% der wiederkehrenden Struktureinheit der Formel (4) 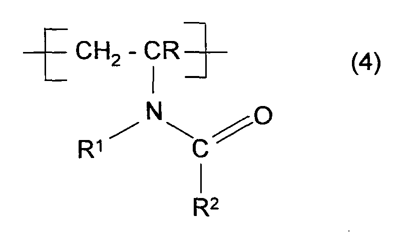 wobei R, R¹ und R² gleich oder verschieden sein können und Wasserstoff oder eine lineare oder verzweigte Alkyl- oder Alkenylgruppe mit jeweils 1 bis 30, bevorzugt 1 bis 20, insbesondere 1 bis 12 C-Atomen, bedeuten
oder
a3) 1 bis 50 Gew.-% einer Mischung der wiederkehrenden Struktureinheit der Formel (3) und der wiederkehrenden Struktureinheit der Formel (4)
und
b) 49,99 bis 98,99 Gew.-% der wiederkehrenden Struktureinheit der Formel (2) 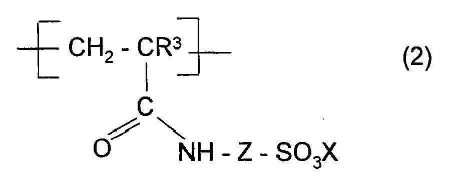 worin
R³ für Wasserstoff, Methyl oder Ethyl steht,
Z für (C₁-C₈)-Alkylen steht, und
X für Wasserstoff, Lithium, Natrium, Kalium, Magnesium, Calcium, Ammonium, Monoalkylammonium, Dialkylammonium, Trialkylammonium oder Tetraalkyl-ammonium, wobei die Alkylsubstituenten der Ammoniumionen unabhängig voneinander (C₁-C₂₂)-Alkylreste darstellen, die mit 0 bis 3 Hydroxyalkylgruppen besetzt sein können, deren Alkylkettenlänge in einem Bereich von C₂ bis C₁₀ variieren kann, steht oder X auch für ein bis dreifach ethoxylierte Ammoniumverbindungen mit gleichem oder unterschiedlichem Ethoxylierungsgrad steht, und wobei in den Copolymeren auch Struktureinheiten der Formel (2) mit unterschiedlichem X enthalten sein können,
und
c) 0 bis 8 Gew.-% an vernetzenden Strukturen, die aus Monomeren mit mindestens zwei olefinischen Doppelbindungen hervorgehen.

17. Zubereitung nach einem oder mehreren der Ansprüche 1 bis 16, **dadurch gekennzeichnet, dass** der oder die wasserlöslichen oder wasserquellbaren vernetzten oder unvernetzten copolymeren oder homopolymeren Verdicker der Komponente II) ausgewählt sind aus vernetzten Polymeren, die vernetzende Strukturen hervorgegangen aus Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate, bevorzugt Trimethylolpropantriacrylat (TMPTA) enthalten.

18. Zubereitung nach einem oder mehreren der Ansprüche 1 bis 17, **dadurch gekennzeichnet, dass** der oder die wasserlöslichen oder wasserquellbaren vernetzten oder unvernetzten copolymeren oder homopolymeren Verdicker der Komponente II) ausgewählt sind aus vernetzten Polymeren, die vernetzende Strukturen hervorgegangen aus Trimethylolpropantriacrylat enthalten.

19. Zubereitung nach einem oder mehreren der Ansprüche 1 bis 18, **dadurch gekennzeichnet, dass** die ein oder mehreren wasserlöslichen unvernetzten Copolymere der Komponente I) und/oder die ein oder mehreren wasserlöslichen oder wasserquellbaren vernetzten oder unvernetzten copolymeren oder homopolymeren Verdicker der Komponente II) ein oder mehrere weitere Struktureinheiten hervorgegangen aus einem oder mehreren Monomeren ausgewählt aus olefinisch ungesättigten Säuren und deren Salzen mit ein- und zweiwertigen Gegenionen, beispielsweise Styrolsulfonsäure, Vinylsulfonsäure, Vinylphosphonsäure, Allylsulfonsäure, Methallylsulfonsäure, Acrylsäure, (Meth)acrylsäure, Maleinsäure bzw. Maleinsäureanhydrid und deren Salze; Ester der Acryl- und der (Meth)acrylsäure mit aliphatischen, aromatischen oder cyclo-aliphatischen Alkoholen mit einer Kohlenstoffzahl von 1 bis 22; Ester der Acryl- und der (Meth)acrylsäure mit Alkylethoxylaten, offenkettige und cyclische N-Vinylamide (N-Vinyllactame) mit einer Ringgröße von 4 bis 9 Atomen, besonders bevorzugt N-Vinylformamid (NVF), N-Vinylmethylformamid, N-Vinyl-methylacetamid (VIMA), N-Vinylacetamid, N-Vinylpyrrolidon (NVP) und N-Vinylcaprolactam; Amide der Acryl- und der Methacrylsäure, besonders bevorzugt Acrylamid, N,N-Dimethylacrylamid, N,N-Diethylacrylamid, alkoxylierte Acryl- und Methacrylamide, wie z.B. MAPTAC und APTAC; 2-Vinylpyridin; 4-Vinylpyridin; Vinylacetat; Methacrylsäureglycidylester; Acrylnitril; Vinylchlorid; Vinylidenchlorid; Tetrafluorethylen und/oder DADMAC, enthalten.

20. Zubereitung nach einem oder mehreren der Ansprüche 1 bis 19, **dadurch gekennzeichnet, dass** das Gewichtsverhältnis der unvernetzten Polymere der Komponente I), vorzugsweise der entsprechenden unvernetzten hydrophob modifizierten Polymere der Komponente I), zu dem einem oder den mehreren wasserlöslichen oder wasserquellbaren polymeren Verdickern der Komponente II) im Bereich von 1 bis 99 zu 99 bis 1, bevorzugt im Bereich von 10 bis 90 zu 90 bis 10, besonders bevorzugt im Bereich von 20 bis 80 zu 80 bis 20, und insbesondere bevorzugt im Bereich von 30 bis 70 zu 70 bis 30 liegt.

21. Zubereitung nach einem oder mehreren der Ansprüche 1 bis 20, **dadurch gekennzeichnet, dass** sie die Polymer-Mischung der Komponenten I) und II) in einer Menge von 0,1 bis 10 Gew.-% enthält.

22. Zubereitung nach einem oder mehreren der Ansprüche 1 bis 21, **dadurch gekennzeichnet, dass** sie in Form eines Haarbehandlungs-, Haarpflege-, Haarstyling- oder Haarreinigungsmittels vorliegt.

23. Zubereitung nach einem oder mehreren der Ansprüche 1 bis 22, **dadurch gekennzeichnet, dass** sie in Form eines wässrigen, gelförmigen kosmetischen oder pharmazeutischen Mittels vorliegt.

24. Zubereitung nach einem oder mehreren der Ansprüche 1 bis 23, **dadurch gekennzeichnet, dass** sie in Form eines Haargels vorliegt und vorzugsweise ein klares, transparentes oder durchscheinendes, farbloses Mittel ist.

25. Zubereitung nach einem oder mehreren der Ansprüche 1 bis 24, **dadurch gekennzeichnet, dass** es in einer versprühbaren Form vorliegt.

26. Zubereitung nach einem oder mehreren der Ansprüche 1 bis 25, **dadurch gekennzeichnet, dass** es einen oder mehrere Filmbildner enthält.

27. Zubereitung nach einem oder mehreren der Ansprüche 1 bis 26, **dadurch gekennzeichnet, dass** sie ein oder mehrere UV-Filter enthält.

28. Zubereitung nach einem oder mehreren der Ansprüche 1 bis 27, **dadurch gekennzeichnet, dass** sie ein oder mehrere Antioxidatien enthält.

29. Zubereitung nach einem oder mehreren der Ansprüche 1 bis 28, **dadurch gekennzeichnet, dass** sie klar transparent oder durchscheinend ist.

30. Zubereitung nach einem oder mehreren der Ansprüche 1 bis 29, **dadurch gekennzeichnet, dass** sie emulgator- und/oder ölfrei ist.

31. Zubereitung nach einem oder mehreren der Ansprüche 1 bis 30, **dadurch gekennzeichnet, dass** sie als Komponente II) eine oder mehrere wasserlösliche oder wasserquellbare vernetzte copolymere Verdicker enthaltend ein oder mehrere Struktureinheiten der Formel (1), ein oder mehrere Struktureinheiten der Formel (2) und ein oder mehrere vernetzende Struktureinheiten, die aus Monomeren mit mindestens zwei olefinischen Doppelbindungen hervorgehen, enthalten.
